# FASCICULE DE BREVET EUROPEEN

(11) **EP 2 844 305 B1**
(45) Date de publication et mention de la délivrance du brevet: **06.01.2016**
(21) Numéro de dépôt: 13719854.5
(22) Date de dépôt: 02.05.2013
(51) Int. Cl.: A61L 2/10, A61L 2/24, B29C 33/72, B29C 49/42

(54) **INSTALLATION POUR LA FABRICATION DE RÉCIPIENTS À PARTIR DE PRÉFORMES COMPORTANT UN DISPOSITIF DE DÉCONTAMINATION DE MOULE ET PROCÉDÉ DE DÉCONTAMINATION**
VORRICHTUNG ZUR HERSTELLUNG VON BEHÄLTERN AUS VORFORMEN UND MIT EINER FORMDEKONTAMINIERUNGSVORRICHTUNG SOWIE DEKONTAMINIERUNGSVERFAHREN
EQUIPMENT INTENDED FOR MANUFACTURING CONTAINERS FROM PREFORMS AND COMPRISING A MOLD-DECONTAMINATING DEVICE, AND DECONTAMINATION METHOD

(30) Priorité: 04.05.2012 FR 1254123
(43) Date de publication de la demande: 11.03.2015
(73) Titulaire: Sidel Participations, 76930 Octeville-sur-Mer (FR)
(72) Inventeur: QUETEL, François, F-76930 Octeville-sur-Mer (FR); DEMARE, Jérôme, F-76930 Octeville-sur-Mer (FR); LETELLIER, Sandy, F-76930 Octeville-sur-Mer (FR); YGER, Benjamin, F-76930 Octeville-sur-Mer (FR)
(86) Numéro de dépôt international: PCT/EP2013/059109
(87) Numéro de publication internationale: WO 2013/164387

(56) Documents cités:
- DE-A1- 4 407 183
- US-A1- 2010 089 009
- US-A1- 2011 037 187
- US-A1- 2011 061 690
- US-A1- 2011 305 597
- US-A1- 2011 311 675
- US-B2- 7 703 262

## Description

L'invention concerne une installation pour la fabrication de récipients à partir de préformes comportant un dispositif de décontamination de moule et un procédé de décontamination.

L'invention concerne plus particulièrement une installation pour la fabrication de récipients à partir de préformes en matière thermoplastique, ladite installation comportant au moins :
- une machine de moulage qui comporte au moins un dispositif de moulage comportant un moule ayant un axe principal, ledit moule comportant au moins deux éléments de moule pourvus chacun d'une surface de moulage et qui sont montés mobiles entre une position ouverte et une position fermée du moule, et
- un dispositif de décontamination d'au moins un moule porté par un dispositif de moulage de la machine, ledit dispositif de décontamination associé à la machine comportant au moins des moyens d'irradiation à rayonnement ultraviolet.

On connaît de l'état de la technique de tels moules qui sont utilisés pour la fabrication de récipients, notamment la fabrication de bouteille en matière thermoplastique comme le PET (Polyéthylène téréphtalate).

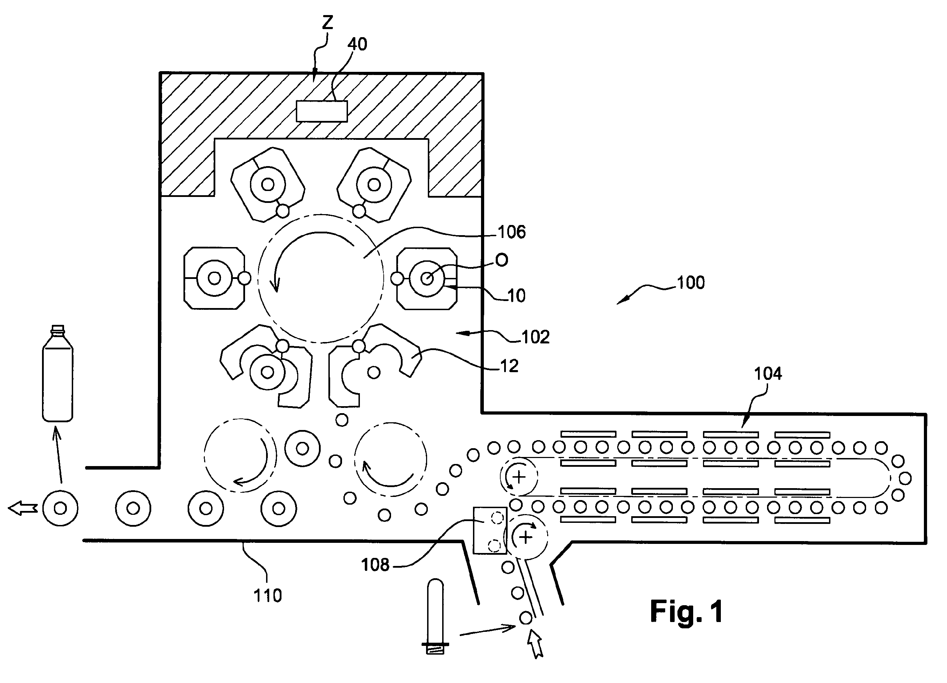

La figure 1 représente schématiquement, à titre d'exemple non limitatif, une installation de fabrication de récipients.

Une installation de fabrication identique est notamment décrite et représentée dans le document EP-A1-2.292.406 auquel on se reportera pour une description détaillée de l'ensemble.

L'installation 100 de fabrication de récipients comporte une machine 102 de moulage (encore appelée "souffleuse") qui est associée à un four 104 pour le conditionnement thermique des préformes.

La machine 102 comporte un carrousel 106 pourvu circonférentiellement d'un nombre "N" de postes formés respectivement par un dispositif 10 de moulage (ou unité de moulage) comportant un moule 12 de fabrication d'au moins un récipient.

Un tel dispositif 10 de moulage (représenté à la figure 2) est similaire à celui qui est décrit et représenté dans le document EP-A1-2.292.406 auquel on se reportera pour de plus amples détails quant à sa structure et son fonctionnement.

Une préforme en matière thermoplastique est successivement conditionnée thermiquement dans le four 104 avant d'être introduite dans le moule 12 de l'un des dispositifs 10 de moulage où la préforme est alors transformée en récipient, notamment par injection d'au moins un fluide sous pression dans la préforme.

Avantageusement, une telle installation 100 comporte encore une machine de remplissage (non représentée), située en aval de la machine 102, dans laquelle les récipients fabriqués sont immédiatement remplis puis fermés, généralement au moyen d'un bouchon.

Dans le domaine de la fabrication de récipients pour l'industrie agro-alimentaire, on recherche par tous moyens à réduire les risques de contaminations microbiologiques des récipients par des agents pathogènes.

C'est la raison pour laquelle, la Demanderesse a déjà proposé de mettre en oeuvre différentes actions pour éliminer des agents pathogènes, tels que les germes (bactéries, moisissures, etc.), qui sont susceptibles d'affecter le produit alimentaire contenu dans les récipients.

Les documents de l'état de la technique cités ci-après et auxquels on se reportera pour de plus amples détails, illustrent à titre d'exemples non limitatifs de telles actions pour lesquelles on peut distinguer d'une part les actions visant à détruire les agents pathogènes et, d'autre part, les actions visant à prévenir la contamination des récipients par de tels agents.

Le document WO-2006/136498 décrit par exemple un traitement de décontamination d'une préforme consistant à déposer par condensation un film de buée sensiblement uniforme d'un agent stérilisant sur la paroi interne de la préforme.

Avantageusement, la décontamination de la préforme est réalisée au moyen d'un dispositif 108 de traitement intervenant avant l'introduction de la préforme dans le four 104.

Un tel traitement est destiné à détruire les agents pathogènes pour décontaminer au moins l'intérieur de la préforme correspondant à la partie dite « alimentaire » du récipient en devenir, c'est-à-dire celle qui sera, après remplissage, en contact direct avec le produit.

Le document FR-2.915.127 décrit une installation de fabrication de récipients comportant une enceinte de protection délimitant une zone à l'intérieur de laquelle est agencée une machine de moulage de récipients de type souffleuse qui est alimentée par des moyens de transfert en préformes préalablement conditionnées thermiquement dans un four.

Selon les enseignements de ce document, l'installation comporte un système d'insufflation d'air filtré à l'intérieur de l'enceinte pour y établir notamment une surpression de manière à limiter les risques de contamination des préformes en sortie du four comme des récipients fabriqués.

L'installation 100 représentée à la figure 1 comporte avantageusement une telle enceinte 110 associée à un système (non représenté) d'insufflation d'air filtré, de tels moyens ayant pour but de prévenir la contamination de l'environnement de fabrication par des agents pathogènes.

Si ce document décrit des risques particuliers de contaminations aéroportées, il existe d'une manière générale un risque de contamination par des agents pathogènes qui, étant présents dans l'environnement de la machine 102 de moulage, sont susceptibles d'être transportés par l'air et de contaminer ainsi les préformes dans le four comme les récipients fabriqués dans la machine de remplissage.

On parle encore de risques de « contaminations croisées » pour désigner plus spécifiquement, dans la machine de moulage, le risque de contamination par des agents pathogènes de l'intérieur de la préforme issue du four, lesdits agents pathogènes aéroportés étant alors susceptibles de contaminer l'intérieur de la préforme avantageusement décontaminé chimiquement.

Le document FR-2.940.964 propose des moyens perfectionnés pour l'escamotage d'une roue de transfert intervenant entre la sortie de la machine de moulage (souffleuse) et une machine de remplissage de manière à limiter certains risques de contamination.

Ce document enseigne plus particulièrement les risques de contamination qui sont inhérents à toute intervention humaine (opérateurs) dans l'environnement immédiat des machines d'une installation de fabrication de récipients, par exemple pour de la maintenance mais d'une manière générale quel que soit le but de l'intervention.

Bien entendu, ces différents exemples d'actions sont avantageusement susceptibles d'être mis en oeuvre en combinaison dans une même installation pour réduire de manière drastique les risques de contamination.

Au cours du processus de fabrication, des agents pathogènes présents dans l'environnement direct des préformes et/ou des récipients, depuis l'air jusqu'aux différents moyens de fabrication de l'installation, sont susceptibles de contaminer directement ou indirectement les préformes et/ou des récipients.

Par conséquent, indépendamment des résultats obtenus notamment avec les actions précitées visant à prévenir la contamination ou à réaliser une décontamination, on recherche encore et toujours à les améliorer.

Or, les agents pathogènes notamment présents sur les surfaces de moulage d'un moule sont susceptibles de contaminer par transfert la surface externe du récipient fabriqué et dès lors, le récipient ainsi contaminé (porteur d'agents pathogènes) devient un vecteur de contamination de l'installation.

Le récipient contaminé est en particulier susceptible d'introduire de tels agents dans l'unité de remplissage qui est la partie la plus sensible de l'installation aux risques de contamination et doit demeurer un environnement stérile.

Plus généralement, la seule présence d'agents pathogènes sur les éléments de moule induit un risque de contamination, c'est-à-dire une présence tant sur les surfaces de moulage que sur les surfaces planes adjacentes déterminant en position fermée le plan de joint du moule.

Il existe alors par conséquent un risque de contamination du volume intérieur du récipient ou de la préforme selon le risque de « contaminations croisées » précité, c'est-à-dire le risque que des agents pathogènes pénètrent à l'intérieur de la préforme ou du récipient.

Le document US-2010/0089009 décrit l'utilisation de moyens d'irradiation, tels que des lampes à rayonnement ultraviolet, pour stériliser notamment une partie d'une machine de moulage de récipients à partir de préformes en matière plastique et également l'intérieur de la cavité du moule monté dans l'un des dispositifs de moulage équipant ladite machine de moulage.

Une telle solution n'est cependant pas satisfaisante pour obtenir une décontamination réellement efficace de l'intérieur du moule.

Par conséquent, les éléments de moule et tout particulièrement leurs surfaces de moulage ne font à ce jour l'objet d'aucun traitement particulier satisfaisant avec les solutions connues de l'état de la technique.

De plus, les éléments de moule sont changés manuellement par un ou des opérateurs, parfois fréquemment, au gré notamment des changements de fabrication d'un récipient à un autre et cela sans que ne soient prises des mesures particulières pour en éliminer les agents pathogènes.

Il existe par conséquent également un risque de contamination directement lié à la présence humaine dans l'environnement de la machine 102 de moulage en vue d'intervenir sur les moules 12.

Le but de la présente invention est notamment de réduire encore les risques de contamination associés aux moules de fabrication de récipients à partir de préformes en matière thermoplastique.

Dans ce but, l'invention propose une installation pour la fabrication de récipients à partir de préformes en matière thermoplastique du type décrit précédemment, caractérisée en ce que ledit dispositif de décontamination comporte des moyens d'entraînement desdits moyens d'irradiation à rayonnement ultraviolet pour introduire lesdits moyens d'irradiation entre lesdits au moins deux éléments de moule constitutifs du moule du dispositif de moulage, lesdits moyens d'entraînement étant commandés pour déplacer lesdits moyens d'irradiation par rapport à la machine entre au moins :
- une position de repos dans laquelle les moyens d'irradiation sont escamotés pour éviter toute interférence entre lesdits moyens d'irradiation du dispositif de décontamination et le moule du dispositif de moulage de la machine, et au moins
- une position de travail dans laquelle les moyens d'irradiation sont agencés à l'intérieur du moule occupant sa position ouverte, entre lesdits éléments du moule pour décontaminer par irradiation au moins lesdites surfaces de moulage.

Avantageusement, lesdits moyens d'irradiation sont montés mobiles par rapport au moule à décontaminer et sont déplacés afin d'être introduits sélectivement à l'intérieur du moule en position ouverte de manière à décontaminer alors l'ensemble de la surface intérieure desdits au moins deux éléments de moule et tout particulièrement mais non exclusivement lesdites surfaces de moulage.

Les moyens d'irradiation sont aptes à irradier également les surfaces planes des éléments de moule qui, adjacentes auxdites surfaces de moulages, forment le plan de joint du moule en position fermée.

Avantageusement, les moyens d'irradiation sont aptes à irradier la bague de col du moule comportant une surface horizontale plane sur laquelle, en production, prennent appui la préforme par l'intermédiaire de sa collerette et une partie des moyens de transformation de la préforme par soufflage ou par étirage soufflage (ou tuyère), notamment des moyens d'étanchéité.

L'invention propose, après avoir identifiés les risques de contaminations associés aux surfaces de moulage des éléments de moule, une solution simple, fiable et économique pour éliminer ces risques et ce faisant améliorer encore l'hygiène au cours de la fabrication de récipients.

Grâce au dispositif de décontamination selon l'invention, le rayonnement ultraviolet est émis par les moyens d'irradiation à proximité immédiate de la surface des au moins deux éléments de moule, et tout particulièrement des surfaces de moulage, grâce à quoi l'effet germicide est optimal et la décontamination est obtenue en tout point desdits au moins deux éléments de moule.

Seul l'agencement des moyens d'irradiation à proximité des surfaces des éléments de moule permet d'obtenir une décontamination efficace, il est par conséquent essentiel d'introduire lesdits moyens d'irradiation, de préférence la source elle-même, directement entre les éléments de moule en position ouverte.

Par comparaison dans le document US-2010/0089009, les moyens d'irradiation demeurent à l'extérieur et émettent seulement un rayonnement ultraviolet en direction du moule se trouvant en position ouverte de sorte que la quantité de rayonnement UV reçue par les surfaces de moulage du moule est très limitée et insuffisante pour prétendre obtenir une décontamination efficace.

De plus, la surface de moulage façonnant l'aspect externe d'un récipient, tel qu'une bouteille, est souvent ouvragée pour des raisons esthétiques (décors en relief, etc.) et/ou fonctionnelles (compression du récipient après utilisation, résistance mécanique aux chocs, etc.).

L'agencement proximal des moyens d'irradiation par rapport à la surface de moulage de chaque élément de moule permet d'irradier totalement, pendant une durée déterminée, lesdites surfaces, en tout point c'est à dire sans que ne subsiste la moindre zone d'ombre non irradiée.

De plus, en considération de l'énergie de rayonnement ultraviolet émis par les moyens d'irradiation et reçu notamment par la surface des éléments de moule, on obtient une efficacité maximale.

Par comparaison, les moyens d'irradiation selon le document US-2010/0089009 ne peuvent irradier l'ensemble des surfaces de moulage en raison notamment de la faible ouverture des éléments de moulage.

Le rayonnement ultraviolet étant émis depuis l'extérieur et à distance du moule, une partie des surfaces de moulage ne sont pas irradiées car elles demeurent dans l'ombre. Compte tenu de l'agencement de la source de rayonnement, une partie du dispositif de moulage comprenant le moule forme un écran qui, arrêtant le rayonnement ultraviolet, conduit à l'apparition d'un phénomène de zones d'ombres et cela tout particulièrement pour la surface de moulage en creux dans le moule.

Ce problème de phénomène d'ombres se produit également plus localement au niveau des surfaces de moulage lorsque celles-ci sont ouvragées par rapport au récipient à fabriquer.

Avantageusement, l'introduction selon l'invention des moyens d'irradiation à l'intérieur du moule permet de résoudre ce problème de zones d'ombre survenant avec l'état de la technique et la totalité de la surface de moulage est décontaminée.

Avantageusement, le déplacement relatif des moyens d'irradiation en position de travail à l'intérieur du moule, en particulier pour effectuer un balayage vertical, fait varier l'angle d'incidence du rayonnement ultraviolet rentrant en contact avec lesdites surfaces et ce faisant permet de parfaire encore l'irradiation des surfaces de moulage même lorsque ces dernières sont ouvragées.

Avantageusement, le dispositif de décontamination selon l'invention permet de réaliser automatiquement au moins une étape de décontamination d'au moins lesdites surfaces de moulage grâce à quoi on évite toute intervention humaine telle que celle d'un opérateur au voisinage des éléments de moule comportant lesdites surfaces de moulage.

Plus généralement, grâce à l'automatisation des différentes opérations nécessaires pour opérer un changement de moule, on supprime l'ensemble des risques de contamination associés à la présence d'un tel opérateur.

En effet et tel qu'expliqué précédemment, un opérateur constitue un vecteur potentiel important d'introduction d'agents pathogènes, en particulier de contamination des surfaces des éléments de moule dont celles de moulage.

Par conséquent, la décontamination est avantageusement réalisée sur au moins un moule monté dans le dispositif de moulage d'une machine de moulage d'une installation de fabrication de récipients à partir de préformes.

Avantageusement, le dispositif de décontamination est mis en oeuvre pour traiter successivement les moules d'une machine de moulage (souffleuse) lorsque ladite machine n'est pas en mode de fonctionnement, dit de production, et tout particulièrement mais non exclusivement après des opérations de changement des moules.

Selon d'autres caractéristiques de l'invention :
- lesdits moyens d'entraînement déplacent dans l'espace selon un trièdre (X, Y, Z) lesdits moyens d'irradiation pour les introduire, orthogonalement par rapport à l'axe principal du moule, à l'intérieur du moule en position ouverte ;
- le dispositif de moulage de la machine comportant ledit moule en position ouverte occupe une position fixe par rapport au dispositif de décontamination au cours d'un cycle de décontamination par lesdits moyens d'irradiation ;
- lesdits moyens d'entraînement comportent au moins un actionneur pour déplacer lesdits moyens d'irradiation transversalement selon l'axe Y du trièdre (X, Y, Z) entre la position de repos et une position intermédiaire qui est déterminée par rapport à un axe principal du moule ;
- lesdits moyens d'entraînement comportent au moins un actionneur pour déplacer lesdits moyens d'irradiation longitudinalement selon l'axe X du trièdre (X, Y, Z) entre la position de repos ou ladite position intermédiaire et ladite position de travail ;
- lesdits moyens d'entraînement comportent au moins un actionneur pour déplacer lesdits moyens d'irradiation verticalement selon l'axe Z du trièdre (X, Y, Z), notamment pour réaliser un balayage vertical des surfaces de moulage du moule ;
- des moyens de positionnement sont respectivement disposés sur le moule et/ou sur le dispositif de décontamination pour déterminer la position relative des moyens d'irradiation par rapport au moule et piloter lesdits moyens d'entraînement associés aux moyens d'irradiation à rayonnement ultraviolet ;
- lesdits moyens d'irradiation sont activés au moins en position de travail ;
- les moyens d'irradiation comportent entre eux un logement destiné à être traversé verticalement par une tige d'étirage du dispositif de moulage qui est associée audit moule de manière à décontaminer au moins une partie de ladite tige d'étirage avec lesdits moyens d'irradiation ;
- des moyens réflecteurs sont associés auxdits moyens d'irradiation pour focaliser le rayonnement ultraviolet émis en direction au moins desdites surfaces de moulage ;
   -- les moyens d'irradiation constitue une tête qui présente une forme complémentaire de l'espace interne délimité par lesdits au moins deux éléments de moule de manière à introduire et positionner lesdits moyens d'irradiation à proximité au moins desdites surfaces de moulage ;
   -- les moyens d'irradiation sont disposés sur le dispositif de décontamination de manière à émettre verticalement vers le bas un rayonnement ultraviolet en direction d'une portion de surface de moulage en vis-à-vis ou d'une portion de la surface d'appui adjacente à une ouverture pour la préforme que présente les éléments de moule ;
- le dispositif de décontamination comporte des moyens de nettoyage qui sont commandés sélectivement pour nettoyer au moins lesdites surfaces de moulage.

L'invention concerne encore un procédé de décontamination au moyen d'un dispositif de décontamination comportant au moins des moyens d'irradiation à rayonnement ultraviolet consistant à décontaminer au moins un moule d'axe principal qui est porté par un dispositif de moulage équipant une machine de moulage d'une installation de fabrication de récipients à partir de préforme en matière thermoplastique et qui est monté mobile entre une position ouverte et une position fermée du moule, caractérisé en ce que ledit procédé de décontamination comporte au moins successivement :
- une étape (b0) de positionnement consistant à commander des moyens d'entraînement associés aux moyens d'irradiation à rayonnement ultraviolet pour introduire, orthogonalement à l'axe principal du moule, lesdits moyens d'irradiation entre lesdits au moins deux éléments du moule occupant ladite position ouverte du moule ;
- une étape (b1) de décontamination consistant à irradier au moins lesdites surfaces de moulage desdits éléments du moule avec lesdits moyens d'irradiation à rayonnement ultraviolet.

De préférence, le procédé de décontamination comporte, avant lesdites étapes (b0) et (b1), une étape (a) de nettoyage consistant au moins à appliquer un agent nettoyant sur au moins lesdites surfaces de moulage du moule pour obtenir un moule propre.

Avantageusement, l'étape (a) de nettoyage consiste à mouiller au moins lesdites surfaces de moulage, au moyen dudit agent nettoyant à l'état liquide et à frotter au moins lesdites surfaces desdits éléments du moule de moulage pour obtenir un moule propre qui est ensuite décontaminé.

De préférence, le procédé de décontamination détermine un cycle de décontamination d'un moule qui est mis en oeuvre pour traiter successivement au moins une partie des moules d'une machine de moulage lorsque ladite machine n'est pas en mode de fonctionnement, dit de production, soit le mode dans lequel des récipients sont fabriqués à partir de préforme en matière thermoplastique.

Avantageusement, la fabrication de récipients par la machine de moulage de l'installation est interrompue pour mettre en oeuvre au moins un cycle de décontamination de tout ou partie des moules de la machine, par exemple après une temps de fabrication donné ou encore une quantité déterminée de récipients fabriqués.

De préférence, le cycle de décontamination réalisé avec le dispositif de décontamination selon l'invention est mis en oeuvre systématiquement lors d'un changement de moule sur la machine de moulage et ceci pour décontaminer au moins les nouveaux moules montés avant le lancement d'une nouvelle fabrication de récipients.

Avantageusement, le cycle de décontamination est également mis en oeuvre pour décontaminer les moules destinés à être changés, avant leur stockage dans un magasin par exemple.

D'autres caractéristiques et avantages de l'invention apparaîtront à la lecture de la description qui va suivre pour la compréhension de laquelle on se reportera aux dessins annexés dans lesquels :
- la figure 1 est une vue schématique de dessus qui représente partiellement un exemple d'installation de fabrication comportant au moins une machine de moulage et un dispositif de décontamination associé ;
- la figure 2 est une vue en perspective qui représente un exemple de dispositif de moulage comportant un moule de fabrication d'un récipient apte à être monté sur une machine d'une installation selon la figure 1 et qui illustre ledit moule en position ouverte ;

- les figures 3 et 4 sont des vues en perspective qui représentent un mode de réalisation d'un dispositif de décontamination de moule ;
- la figure 5 est une vue en perspective qui représente ensemble un dispositif de décontamination selon les figures 3 et 4 et un moule d'un dispositif de moulage et qui illustre un dispositif de décontamination positionné relativement à un moule en position ouverte et dont les moyens d'irradiation sont en position de repos ;
- la figure 6 est une vue en perspective analogue la figure 5 qui représente ensemble un dispositif de décontamination et un moule en position ouverte et qui illustre les moyens d'irradiation du dispositif de décontamination qui, en position de travail, sont introduits à l'intérieur du moule en position ouverte.

Dans la suite de la description, des éléments similaires ou identiques seront désignés par les mêmes références.

Dans la description, on utilisera à titre non limitatif les expressions telles que "amont" et "aval", "supérieur" et "inférieur", "intérieur" et "extérieur", "avant" et "arrière" selon les définitions données dans la description et les orientations longitudinale, transversale et verticale en référence au trièdre (X, Y, Z) représenté sur les figures.

Tel que décrit précédemment, la figure 1 représente une installation 100 pour la fabrication de récipients à partir de préformes en matière thermoplastique.

L'installation 100 comporte au moins une machine 102 de moulage (ou souffleuse) qui comporte au moins un dispositif 10 de moulage comportant un moule 12 ayant un axe (O) principal.

De préférence, l'axe (O) principal du moule 12 du dispositif 10 de moulage est associé à l'axe principal de la préforme qui est destinée à y être transformée en récipient lorsque la machine 102 de moulage est en mode de fonctionnement, dit de production.

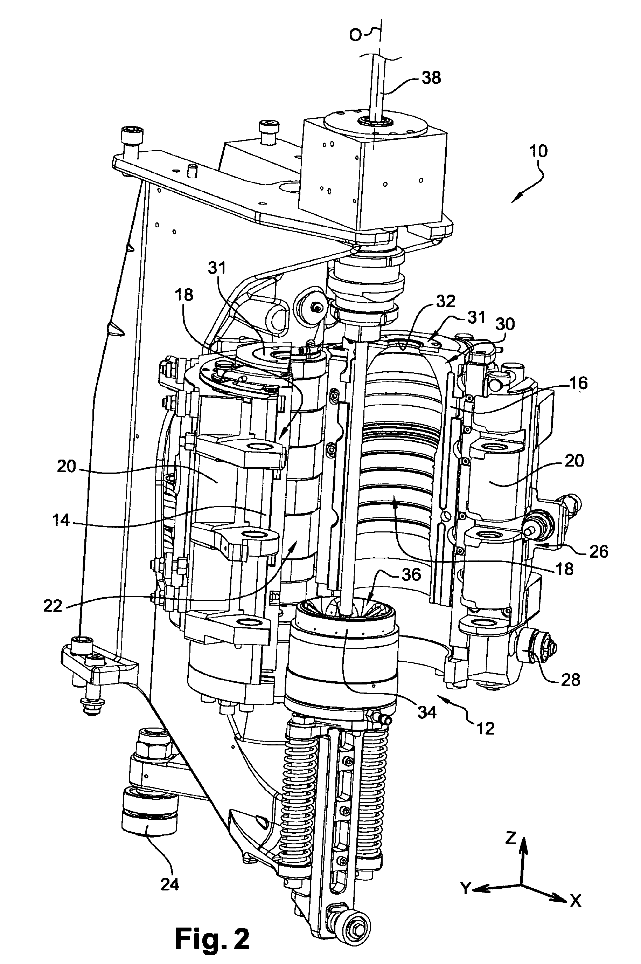

On a représenté en détail à la figure 2 (sans l'ensemble de la machine 102 de moulage), à titre non limitatif, un exemple de réalisation d'un dispositif 10 de moulage apte à équiper la machine 102 de moulage d'une installation 100 de fabrication de récipients selon la figure 1.

Un tel dispositif 10 de moulage est destiné à la fabrication de récipients en matière thermoplastique, en particulier des bouteilles, par soufflage ou étirage-soufflage d'une préforme préalablement conditionnée thermiquement dans un four 104.

Le document FR-2.764.544 décrit et représente des moyens de soufflage ou d'étirage-soufflage qui, non représentés sur la présente figure 2 par simplification, sont destinés à équiper un tel dispositif 10 de moulage.

De tels moyens sont encore parfois appelés et réalisés sous la forme d'une tuyère de soufflage, on se reportera pour de plus amples détails au document donné toutefois uniquement à titre d'exemple non limitatif.

Comme on peut le voir sur la figure 2, le dispositif 10 de moulage comporte principalement un moule 12 comportant au moins deux éléments de moule, respectivement un élément 14 de moule gauche et un élément 16 de moule droit.

Les deux éléments 14, 16 de moule sont montés mobiles l'un par rapport à l'autre entre une position ouverte du moule 12 et une position fermée du moule.

Dans la présente description, on entend par "élément de moule" une pièce du moule 12 comportant une partie de la surface de moulage ou empreinte du récipient à fabriquer et cela indépendamment de la conception du moule 12 du dispositif 10 de moulage.

Dans l'exemple de réalisation, les deux éléments 14, 16 de moule comportent notamment une surface 18 de moulage et sont respectivement fixés de manière démontable par des moyens de fixation au porte-moule 20 qui est associé à chaque élément 14, 16 de moule.

Dans l'exemple représenté, les porte-moules 20 sont constitués sous la forme de deux structures porteuses montées pivotantes autour d'un axe commun de rotation, l'axe de rotation s'étendant ici verticalement selon le trièdre (X, Y, Z).

Chaque porte-moule 20 comporte, selon la direction longitudinale, une partie arrière qui est complémentaire de celle de l'autre porte-moule de manière à s'interpénétrer pour former une articulation 22 de type charnière.

En variante, seulement l'un des porte-moules est monté mobile tandis que l'autre porte-moule est fixe, le porte-moule mobile étant commandé en déplacement entre lesdites positions ouverte ou fermée.

Les porte-moules 20 et donc les éléments 14, 16 de moule sont commandés sélectivement pour s'écarter mutuellement l'un de l'autre en pivotant autour de l'axe de rotation, respectivement entre lesdites positions ouverte et fermée du moule 12.

En raison de sa cinématique, le moule 12 d'un tel dispositif 10 de moulage est encore appelé moule "portefeuille" (*ou "book-like opening" en anglais*).

Le document WO-2004/018181, auquel on se reportera pour de plus amples détails, décrit l'articulation d'un tel moule 12 et des moyens de commande associés pour en provoquer l'ouverture ou la fermeture.

De préférence, l'entraînement entre lesdites positions ouverte et fermée des éléments 14, 16 de moule solidaires des porte-moules 20 est obtenu par un système de bras de traction dont une extrémité est montée articulée sur les porte-moules 20 et dont l'autre extrémité libre comporte par exemple un galet 24 apte à coopérer avec des moyens d'actionnement complémentaires, tels qu'une came, pour commander sélectivement l'ouverture et la fermeture du moule 12.

Le dispositif de moulage 10 comporte encore un verrou 26 qui, agencé longitudinalement en partie avant, soit à l'opposé de l'articulation 22 pivotante, est destiné à assurer le verrouillage du moule 12 en position fermée.

Un tel verrou 26 est également connu et ne sera donc pas décrit plus en détail, ledit verrou 26 étant par exemple commandé au moyen d'un organe 28 et ayant notamment pour fonction de prévenir toute ouverture inopinée lors des opérations de transformation de la préforme par soufflage pour lesquelles la pression finale peut atteindre 40 bars.

Pour de plus amples détails sur la structure et le fonctionnement d'un verrou 26, on pourra par exemple se reporter au document FR-2.646.802.

Bien entendu, il ne s'agit que d'un exemple car il existe de nombreux verrous, différents mais équivalents, susceptibles d'assurer la fonction de verrouillage.

De préférence et tel qu'illustré à la figure 2, le dispositif 10 de moulage est du type dans lequel chaque élément 14, 16 de moule du moule 12 est réalisé selon une conception en deux parties distinctes, respectivement une coquille munie d'une partie de l'empreinte ou surface de moulage du récipient à fabriquer et un porte-coquille destiné à supporter la coquille et apte à être lui-même solidarisé à l'un des porte-moules 20.

Une telle conception du moule 12 présente de nombreux avantages notamment décrits dans le document EP-0.821.641 auquel on se reportera pour plus de détails.

Chaque élément 14, 16 de moule comporte en creux, dans une face interne 30, une partie de ladite surface 18 de moulage du récipient.

Chaque surface 18 de moulage, réalisée en creux dans la face interne 30 de l'élément 14, 16 de moule, est entourée d'une surface plane d'orientation verticale formant le plan de joint du moule lorsque, le moule 12 occupant la position fermée, lesdits éléments 14 et 16 de moule sont réunis en étant accolés l'un à l'autre.

Lorsque le moule 12 est en position fermée, les deux élément 14, 16 de moule réunis ensemble déterminent une face 31 supérieure horizontale présentant centralement une ouverture 32 destinée à permettre l'introduction de la préforme.

La face 31 supérieure horizontale constitue une surface d'appui d'une part pour la collerette radiale de la préforme introduite dans ladite ouverture 32 et, d'autre part, pour des moyens d'étanchéité de la tuyère de soufflage (non représentée).

De préférence, la face 31 appartient à une pièce distincte réalisée en deux parties et respectivement rapportées à fixation sur les éléments 14, 16 de moule, une telle pièce visible sur la figure 2 est encore appelée "bague de col".

Lorsque le récipient à fabriquer présente un fond de forme complexe, en particulier pétaloïde, des problèmes de démoulage peuvent survenir.

C'est la raison pour laquelle, on prévoit alors avantageusement un élément 34 séparé qui, appelé fond de moule, est distinct des éléments 14, 16 de moule de sorte que moule 12 est réalisé en trois éléments.

Ledit élément 34 formant le fond de moule comporte une surface 36 de moulage correspondant au fond du récipient à fabriquer et qui est complémentaire des surfaces 18 de moulage de chaque élément 14, 16 de moule.

De préférence, le dispositif 10 de moulage comporte une tige 38 d'étirage pour étirer la préforme lors de sa transformation en récipient.

La tige 38 d'étirage s'étend verticalement selon l'axe Z du trièdre (X, Y, Z), coaxialement à l'axe principal (O) du moule 12 du dispositif 10 de moulage.

L'installation 100 pour la fabrication de récipients à partir de préformes en matière thermoplastique comporte un dispositif 40 de décontamination de moule qui, tel que représenté sur la figure 1, est associé à la machine 102 de moulage de l'installation. Le dispositif 40 de décontamination est juxtaposé à la machine 102 de moulage.

Le dispositif 40 de décontamination peut être fixe et dans ce cas demeurer en permanence en place à côté de la machine 102 ou encore être monté mobile de manière à pouvoir être déplacé par rapport à la machine 102.

Le dispositif 40 de décontamination de moule est apte à décontaminer sélectivement au moins un moule 12 porté par un dispositif 10 de moulage de la machine 102, tel que le moule 12 qui vient d'être décrit en référence à la figure 2.

Pour ce faire, le dispositif 40 de décontamination comporte au moins des moyens 42 d'irradiation à rayonnement ultraviolet.

De préférence, lesdits moyens 42 d'irradiation sont commandés pour émettre sélectivement ledit rayonnement ultraviolet.

Les moyens 42 d'irradiation sont par exemple formés par des lampes 44 dites « à ultraviolet » (ou UV) aptes à émettre un rayonnement ultraviolet présentant au moins une raie principale ayant une longueur d'onde comprise entre 100 nm et 400 nm.

En variante, les moyens 42 d'irradiation sont par exemple constitués par des composants de type semi-conducteur, tels que des diodes électroluminescentes ou "LED" ou encore par une lampe de type "flash".

De préférence, le rayonnement ultraviolet est un rayonnement de type "C", c'est-à-dire un rayonnement "UVC" qui présente au moins une raie principale ayant une longueur d'onde comprise entre 100 nm et 280 nm.

Avantageusement, le rayonnement ultraviolet "UVC" présente une raie principale ayant une longueur d'onde égale à 254 nm.

L'action décontaminante du rayonnement ultraviolet résulte tout particulièrement de leur capacité à traverser la membrane du micro-organisme pour en altérer l'acide désoxyribonucléique (ou ADN) du noyau, et c'est la raison pour laquelle les rayonnements ultraviolets sont capables, en altérant l'ADN, d'inhiber la mitose du micro-organisme.

Avantageusement, le dispositif 40 de décontamination est apte à introduire sélectivement les moyens 42 d'irradiation entre lesdits éléments 14, 16 et 34 constitutifs du moule 12, lequel moule est monté sur le dispositif 10 de moulage et occupe sa position ouverte.

Les moyens 42 d'irradiation à rayonnement ultraviolet sont introduits à l'intérieur du moule 12 ouvert d'un des dispositifs 10 de moulage qui, par rotation du carrousel 106 de la machine 102, est amené en position globalement en vis-à-vis du dispositif 40 de décontamination avant de s'immobiliser.

Pour des moyens 42 d'irradiation d'une puissance donnée, l'effet germicide du rayonnement ultraviolet sera d'autant plus important que la quantité d'énergie reçue par la surface irradiée sera importante.

Grâce à l'introduction des moyens 42 d'irradiation à l'intérieur du moule 12 en position ouverte, une décontamination aussi efficace que fiable est obtenue du fait de la proximité entre les moyens 42 d'irradiation et la face 30 interne des éléments 14, 16 de moule ou encore de l'élément 34 de fond de moule.

Les moyens 42 d'irradiation sont introduits dans le moule 12 en position ouverte, suivant une direction de déplacement qui est orthogonale à l'axe principal O du moule 12.

Dans le cas d'un moule 12 de type "portefeuille" selon la figure 2, les moyens 42 d'irradiation sont introduits entre les deux éléments de moule 14 et 16 d'une part et l'élément 34 de fond de moule d'autre part, et cela de manière à agencer lesdits moyens 42 d'irradiation à proximité au moins des surfaces 18 de moulage des éléments 14, 16 et de la surface 36 de l'élément 34 de fond de moule pour décontaminer l'ensemble.

Les moyens 42 d'irradiation étant agencés au plus près et en vis-à-vis direct des surfaces 18, 36 de moulage desdits éléments 14, 16 et 34, le rayonnement ultraviolet émis par les lampes 44 formant lesdits moyens 42 d'irradiation va irradier de manière optimale la surface 36 de moulage du fond et les faces 30 internes desdits éléments 14, 16 de moule comportant lesdites surfaces 18, 36 de moulage.

Grâce à la quantité d'énergie de rayonnement ultraviolet reçue notamment par les surfaces 18, 36 de moulage par rapport à une puissance donnée des lampes 44, on obtient une décontamination efficace et dans un laps de temps relativement court.

Avantageusement, le dispositif 40 de décontamination comporte des moyens 46 d'entraînement qui sont associés auxdits moyens 42 d'irradiation.

Lesdits moyens 46 d'entraînement des moyens 42 d'irradiation à rayonnement ultraviolet sont commandés pour introduire lesdits moyens 42 d'irradiation à l'intérieur du moule 12, entre lesdits éléments 14, 16 et 34 du moule 12 que porte un dispositif 10 de moulage de la machine 102.

Un cycle de décontamination d'un moule 12 par les moyens 42 d'irradiation du dispositif 40 de décontamination est donc réalisé sur un moule 12 monté sur la machine 102 et qui occupe sa position ouverte.

Avantageusement, le cycle de décontamination est réalisé en l'absence de préforme ou de récipient dans le moule 12 afin que l'espace délimité par lesdits éléments 14, 16 et 34 du moule 12 soit complètement libre pour y introduire lesdits moyens 42.

Ainsi, la décontamination du moule 12 au moyen du dispositif 40 de décontamination est mise en oeuvre lorsque la machine 102 ne fabrique pas de récipients.

La fabrication de récipients est par exemple interrompue pour réaliser un cycle de décontamination après un temps de fabrication donné ou encore une quantité déterminée de récipients fabriqués ou bien encore après un changement de moule.

S'agissant d'un moule 12 de type portefeuille selon la figure 2, les moyens 42 d'irradiation sont ici introduits entre lesdits éléments 14, 16 et 34 du moule 12 alors que celui-ci occupe la position ouverte.

De préférence, les moyens 42 d'irradiation sont introduits longitudinalement de l'avant vers l'arrière entre lesdits éléments 14, 16 et 34 du moule 12, orthogonalement à l'axe principal O du moule 12.

De préférence, les moyens 46 d'entraînement sont commandés pour déplacer lesdits moyens 42 d'irradiation dans l'espace, c'est à dire selon les trois axes du trièdre (X, Y, Z).

En variante, les moyens d'entraînement sont aptes à déplacer lesdits moyens 42 d'irradiation selon au moins un axe du trièdre (X, Y, Z) formé par l'axe X.

Grâce aux moyens 46 d'entraînement, lesdits moyens 42 d'irradiation, formés ici par les lampes 44, sont montés mobiles par rapport à la machine (102) dont l'un des dispositifs 10 de moulage comporte le moule 12 à décontaminer.

Avantageusement, les moyens 46 d'entraînement sont commandés pour déplacer lesdits moyens 42 d'irradiation entre au moins une position de repos et une position de travail.

De préférence, les moyens 42 d'irradiation sont susceptibles d'occuper un état inactif et un état actif dans lequel un rayonnement ultraviolet est émis par lesdits moyens 42 d'irradiation.

Avantageusement, lesdits moyens 42 d'irradiation sont activés au moins en position de travail.

La position de repos correspond à une position dans laquelle les moyens 42 d'irradiation sont escamotés, notamment pour éviter toute interférence entre les moyens 42 d'irradiation du dispositif 40 de décontamination et un dispositif 10 de moulage de la machine 102, en particulier avec le moule 12.

Les moyens 42 d'irradiation occupent notamment ladite position de repos lorsque la machine 102 est en mode de fonctionnement, dit de fabrication, dans lequel des récipients sont fabriqués à partir de préformes.

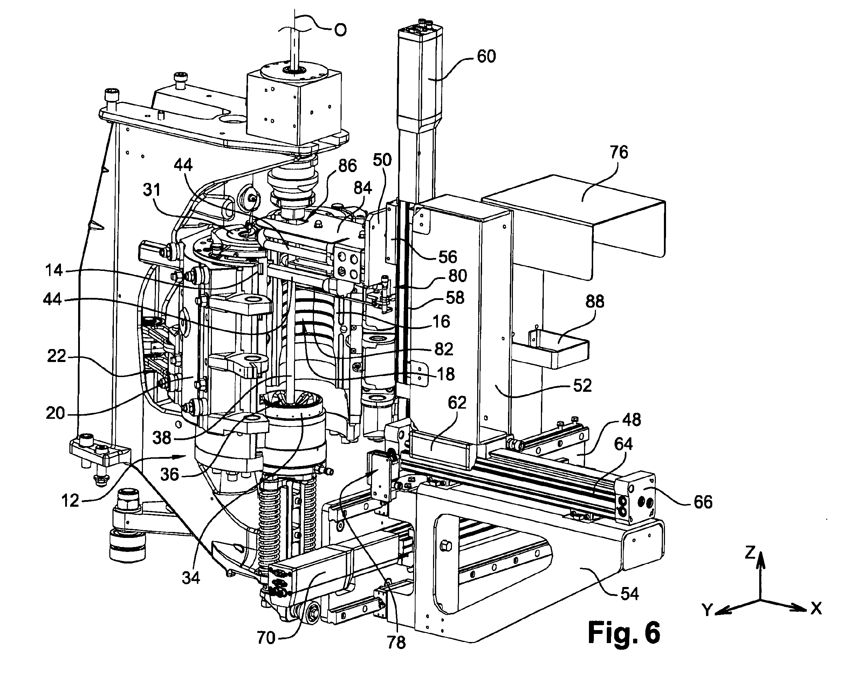

La position de travail correspond à la position, représentée sur la figure 6, dans laquelle lesdits moyens 42 d'irradiation sont introduits entre lesdits éléments 14, 16 et 34 du moule 12 pour décontaminer par irradiation au moins lesdites surfaces 18, 36 de moulage et plus généralement l'ensemble des faces 30 internes pour les éléments 14, 16 de moulage.

En position de travail, les moyens 42 d'irradiation sont donc agencés à l'intérieur du moule 12 occupant sa position ouverte, entre lesdits éléments 14, 16 et 34 du moule 12.

Le dispositif 40 de décontamination comporte un châssis 48 par rapport auquel les moyens 42 d'irradiation sont montés mobiles en trois dimensions suivant le trièdre (X, Y, Z) par l'intermédiaire desdits moyens 46 d'entraînement.

Le châssis 48 comporte au moins une plaque qui s'étend suivant la direction transversale correspondant à l'axe Y du trièdre.

Les moyens 42 d'irradiation sont liés en déplacement à un premier support 50, lequel premier support 50 est monté mobile selon la direction verticale (axe Z) par rapport à un deuxième support 52 intermédiaire.

Le deuxième support 52 intermédiaire est monté mobile selon la direction longitudinale (axe X) par rapport à un troisième support 54, lequel troisième support 54 est monté mobile selon la direction transversale (axe Y) par rapport à la plaque du châssis 48 du dispositif 40 de décontamination.

Grâce aux différents supports 50, 52 et 54 et aux moyens 46 d'entraînement qui leur sont associés, les moyens 42 d'irradiation sont aptes à être déplacés dans l'espace et plus particulièrement déplacés entre lesdites position de repos et de travail.

Le premier support 50 comportant les moyens 42 d'irradiation est monté mobile verticalement par rapport au deuxième support 52 par l'intermédiaire d'un chariot 56 qui est monté coulissant sur un guide 58 associé qui est solidaire du deuxième support 52.

Le chariot 56 est apte à être entraîné sélectivement en déplacement selon l'axe Z par un premier actionneur 60 associé que supporte le deuxième support 52.

De préférence, le premier actionneur 60 est un actionneur électrique qui commande le déplacement du chariot 56 et ce faisant le positionnement vertical, voir le déplacement, des moyens 42 d'irradiation selon l'axe Z.

Le deuxième support 52 est solidaire d'un chariot 62 par l'intermédiaire duquel le deuxième support 52 est monté coulissant longitudinalement sur un guide 64 associé qui est solidaire du troisième support 54.

Le chariot 62 est apte à être entraîné sélectivement en déplacement selon l'axe X par un deuxième actionneur 66 associé que supporte le troisième support 54.

De préférence, le deuxième actionneur 66 est un vérin pneumatique qui commande le déplacement du chariot 62 entre deux positions extrêmes déterminées par des butées.

Le dispositif 40 de décontamination occupant une position déterminée, la distance entre celui-ci et le moule 12 est connue de sorte que le positionnement longitudinal des moyens 42 d'irradiation selon l'axe X est simplifié.

Lorsque le deuxième support 52 coulisse longitudinalement d'avant vers l'arrière et réciproquement, les moyens 42 d'irradiation effectuent alors un déplacement identique suivant l'axe X puisque lesdits moyens 42 d'irradiation sont liés en déplacement au deuxième support 52.

Le troisième support 54 présente globalement une forme d'équerre qui s'étend longitudinalement, orthogonalement à la plaque d'orientation transversale du châssis 48.

Le troisième support 54 est monté mobile longitudinalement par rapport à la plaque du châssis 48 du dispositif 40 de décontamination.

De préférence, les fonctions d'entraînement et de guidage lors du déplacement du troisième support 54 sont assurées par des moyens distincts.

Des moyens 68 de guidage interviennent entre ledit troisième support 54 et la plaque du châssis 48.

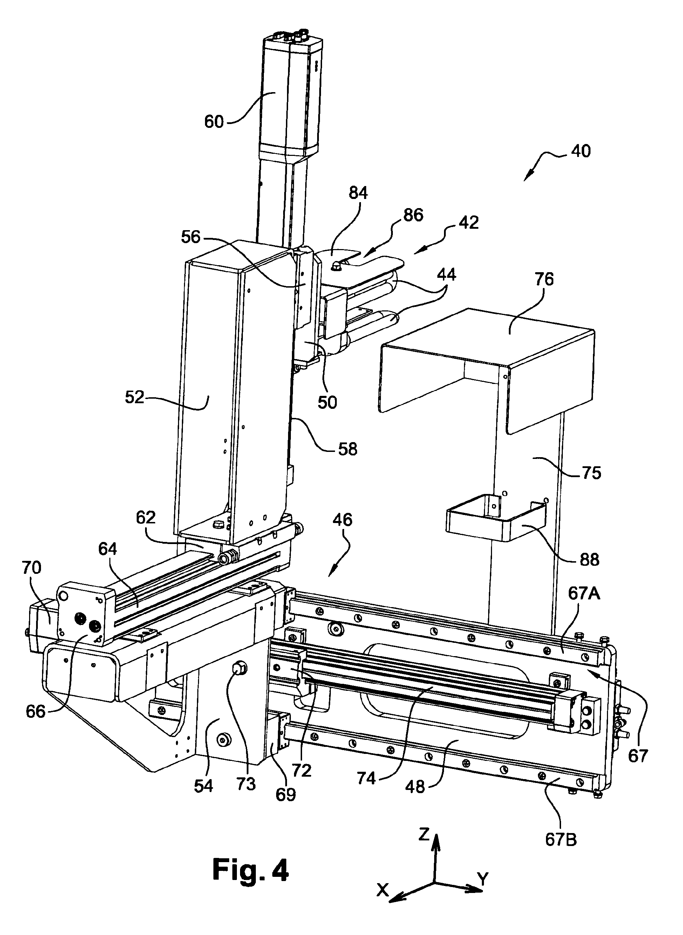

Tel qu'illustré à la figure 4, les moyens 68 de guidage comportent par exemple un rail 67A supérieur et un rail 67B inférieur qui sont solidaires de la plaque du châssis 48 et qui s'étendent transversalement, parallèlement l'un à l'autre.

Le troisième support 54 comporte deux patins 69 qui coulissent respectivement sur le rail 67A supérieur et le rail 67B inférieur lorsque le troisième support 54 est entraîné en déplacement transversalement par rapport à la plaque du châssis 48.

Le déplacement du troisième support 54 est commandé sélectivement grâce à un troisième actionneur 70 qui comporte un chariot 72 visible sur la figure 4.

Le chariot 72 est fixé au troisième support 54 afin de les lier en déplacement selon la direction transversale, par exemple par l'intermédiaire d'une vis 73.

Le chariot 72 coopère avec un guide 74 sur lequel le chariot 72 coulisse transversalement suivant l'axe Y, entraînant alors avec lui en déplacement le troisième support 54 et ce faisant les moyens 42 d'irradiation.

De préférence, le troisième actionneur 70 est un actionneur électrique permettant de faire varier rapidement la vitesse lorsque les moyens 42 d'irradiation sont déplacés de la position de repos vers la position de travail.

Avantageusement, le dispositif 40 de décontamination comporte des moyens 76 de protection des moyens 42 d'irradiation lorsque lesdits moyens 42 occupent ladite position de repos.

De préférence, les moyens de protection comportent au moins un capot 76 en « U » inversé délimitant un logement dans lequel sont reçus lesdits moyens 42 d'irradiation, ledit capot 76 étant fixé à la plaque du châssis 48 par l'intermédiaire d'une pièce 75 de support qui s'étend verticalement.

Avantageusement, le dispositif 40 de décontamination comporte des moyens de positionnement pour déterminer la position relative des moyens 42 d'irradiation par rapport au moule 12 et piloter lesdits moyens 46 d'entraînement associés aux moyens 42 d'irradiation à rayonnement ultraviolet.

Les moyens de positionnement sont respectivement disposés sur le moule 12 et/ou sur le dispositif 40 de décontamination, et sont avantageusement liés en déplacement aux supports 52, 54 et 56.

Les moyens 46 d'entraînement comportent le troisième actionneur 70 pour déplacer lesdits moyens 42 d'irradiation transversalement selon l'axe Y du trièdre (X, Y, Z), grâce à quoi les moyens 42 d'irradiation sont déplacés entre la position de repos et une position intermédiaire qui est déterminée par rapport à l'axe O principal du moule 12.

Pour déterminer ladite position intermédiaire, le dispositif 40 de décontamination comporte des premiers moyens 78 de positionnement selon la direction transversale.

Les premiers moyens 78 de positionnement permettent de piloter le troisième actionneur 70 pour positionner transversalement le deuxième support 54 de manière que les moyens 42 d'irradiation soient centrés par rapport à l'axe O principal du moule 12.

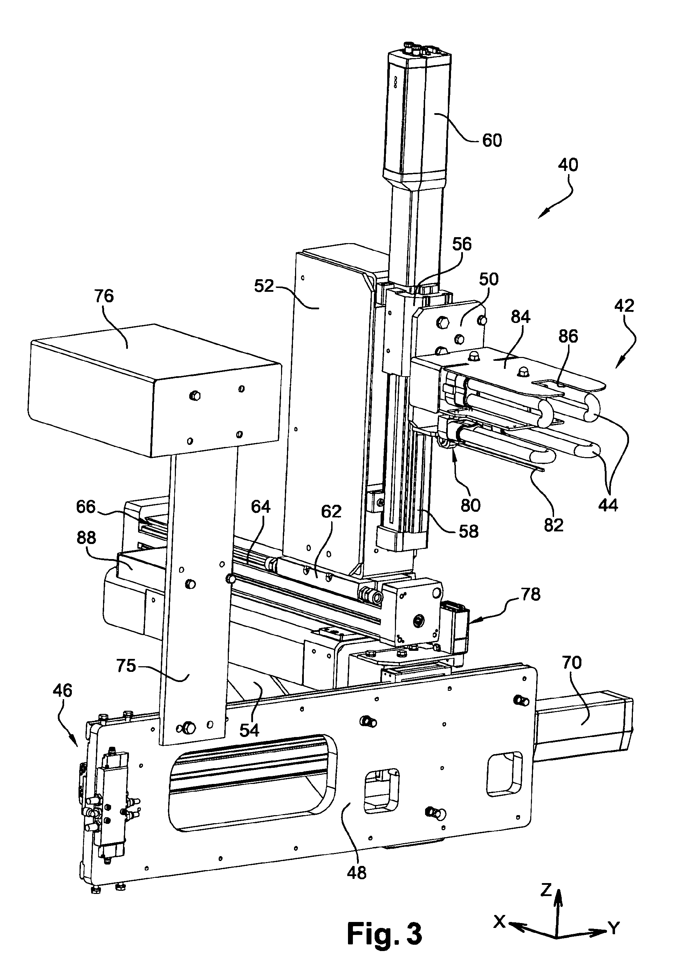

De préférence, les premiers moyens 78 de positionnement sont de type émetteur/récepteur et comportent au moins un émetteur formé par une cellule laser dont le rayon laser R est représenté sur la figure 3 et d'un récepteur associé agencé en dessous dudit émetteur.

De préférence, au moins une cible complémentaire de l'émetteur/récepteur formant les premiers moyens 78 de positionnement est prévue sur le dispositif 10 de moulage comportant le moule 12 pour réfléchir le rayon laser émis.

Toutefois, les premiers moyens 78 de positionnement sont déportés transversalement par rapport aux moyens 42 d'irradiation et la référence de positionnement selon l'axe Y est prise sur l'élément 34 de fond de moule, ladite position intermédiaire des moyens 42 d'irradiation correspondant à la position pour laquelle le rayon laser R lèche la circonférence de l'élément 34 de fond de moule.

Les moyens 46 d'entraînement comportent ensuite le deuxième actionneur 66 pour déplacer lesdits moyens 42 d'irradiation longitudinalement selon l'axe X du trièdre (X, Y, Z), grâce à quoi les moyens 42 d'irradiation sont déplacés entre ladite position intermédiaire et ladite position de travail.

De préférence, les moyens de positionnement selon l'axe X sont intégrés au deuxième actionneur 66 et sont avantageusement réalisés sous la forme de deux butées, respectivement avant et arrière, qui déterminent la course du chariot 62 par rapport au guide 64.

Les moyens 46 d'entraînement comportent enfin le troisième actionneur 60 pour positionner lesdits moyens 42 d'irradiation verticalement selon l'axe Z du trièdre (X, Y, Z), en particulier par rapport à l'élément 34 de fond de moule lorsque le moule 12 présente une conception du type de celle décrite précédemment en référence à la figure 2.

Avantageusement, le troisième actionneur 60 est piloté grâce à des deuxièmes moyens 80 de positionnement.

Dans le mode de réalisation, les moyens 42 d'irradiation comportent par exemple quatre lampes 44 en forme de "U", deux lampes inférieures agencées horizontalement et deux lampes supérieures agencées verticalement et situées verticalement au dessus des deux lampes inférieures.

Les deux lampes 44 inférieures sont notamment, mais non exclusivement, destinées à irradier la surface 36 de moulage de l'élément 34 de fond de moule.

Avantageusement, les lampes 44 formant les moyens 42 d'irradiation sont disposées sur le premier support 50 du dispositif 40 de décontamination de manière à émettre verticalement vers le bas un rayonnement ultraviolet en direction d'une portion de surface de moulage en vis-à-vis, telle que la surface 36 de moulage lorsque le moule 12 comporte un élément 34 de fond de moule.

Les deuxièmes moyens 80 de positionnement ont notamment pour fonction de positionner les moyens 42 d'irradiation de manière que les lampes 44 inférieures soient à proximité de la surface 36 de moulage.

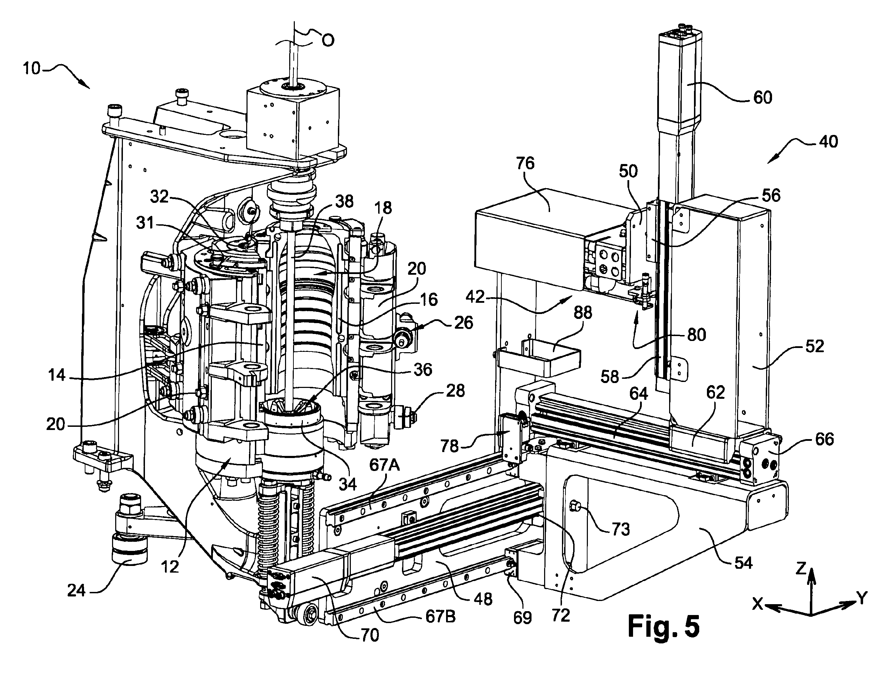

De préférence et tel qu'illustré à la figure 5, les deuxièmes moyens 80 de positionnement sont constitués par un détecteur comportant une tige 82 de détection qui s'étend longitudinalement, une extrémité de la tige 82 étant montée pivotante autour d'un axe transversal sur un support dudit détecteur.

Le support du détecteur est lié en déplacement au premier support 50, ainsi lorsque ledit premier support 50 est déplacé verticalement par le premier actionneur 60, la tige 82 de détection se déplace également.

Après l'introduction des lampes 44 formant les moyens 42 d'irradiation en position de travail entre lesdits éléments 14, 16 et 34 du moule 12, le premier support 50 est déplacé verticalement vers le bas jusqu'à ce que la tige 82 de détection entre en contact avec l'élément 34 de fond de moule.

La tige 82 de détection pivote lorsque son extrémité libre entre en contact avec la partie supérieure de l'élément 34 de fond de moule et le détecteur émet alors un signal grâce auquel le premier actionneur 60 est piloté et la course de descente arrêtée.

Les moyens 42 d'irradiation occupent alors une position de travail, dite position initiale de travail.

Avantageusement, les moyens 42 d'irradiation n'occupent pas une position de travail fixe mais sont déplacés par rapport aux surfaces 18, 36 de moulage du moule 12.

Le troisième actionneur 60 des moyens 46 d'entraînement permet en effet de déplacer verticalement lesdits moyens 42 d'irradiation par rapport aux surfaces 18, 36 de moulage.

Grâce à quoi, les moyens 42 d'irradiation réalisent un balayage des éléments 14, 16, de moule, tout particulièrement des faces 30 comportant lesdites surfaces 18 de moulage du moule 12 en étant déplacé depuis la position de travail initiale (basse) jusqu'à une position de travail finale (haute) représentée sur la figure 6.

Depuis la position de travail initiale, les moyens 42 d'irradiation irradient avantageusement la surface 36 de moulage de l'élément 34 de fond de moule.

Puis, le balayage pour irradier notamment les surfaces 18 de moulage avec le rayonnement ultraviolet est obtenu en commandant le premier actionneur 60 qui fait coulisser le premier support 50 comportant les lampes 44 verticalement vers le haut.

De préférence, lorsque les moyens 42 d'irradiation occupent la position de travail initiale, une temporisation est réalisée pour irradier la surface 36 de l'élément 34 de fond de moule avant de débuter ledit balayage résultant du déplacement vertical vers la position de travail finale.

Avantageusement, en position de travail finale, les moyens 42 d'irradiation sont aptes à irradier la face 31 supérieure horizontale qui, appartenant à la bague de col en deux parties, s'étend autour du logement 32 pour l'introduction de la préforme dans le moule 12 en production.

Avantageusement, on élimine donc également les risques de contamination par des agents pathogènes du col de la préforme, soit celle du récipient en devenir.

Ainsi qu'on l'aura compris, les moyens 42 d'irradiation du dispositif 40 de décontamination sont aptes à décontaminer par ledit rayonnement ultraviolet l'ensemble des surfaces du moule 12 susceptibles de présenter des agents pathogènes et ce faisant d'induire des risques de contamination.

Bien entendu, un tel balayage pourrait n'être pas effectué lorsque les moyens 42 d'irradiation s'étendent verticalement sur une hauteur au moins égale à celle du moule 12 assurant ainsi une irradiation complète de la surface même avec une position de travail fixe.

Toutefois, on comprendra que des moyens 42 d'irradiation de dimension verticale plus réduite soient plus polyvalent pour traiter différents types de moule, s'agissant de la surface de moulage par exemple celle d'un récipient d'une contenance de 2 litres est différente de celle d'un récipient de 0,33 litre.

Avantageusement, un tel balayage tend à renforcer encore l'irradiation totale, en tout point de la surface, tout particulièrement des surfaces 18, 36 de moulage et cela en combinaison l'agencement proximal des moyens 42 d'irradiation par rapport auxdites surfaces.

Avantageusement, les moyens 46 d'entraînement associés aux moyens 42 d'irradiation comportent un premier actionneur 60, un deuxième actionneur 66 et une troisième actionneur 70, grâce auxquels les moyens 42 d'irradiation sont aptes à être déplacés et positionnés avec précision dans l'espace, selon chacune des directions du trièdre (X, Y, Z).

Avantageusement, les lampes 44 à rayonnement ultraviolet formant les moyens 42 d'irradiation du dispositif 40 de décontamination sont agencées sur le premier support 50 de manière à former une tête présentant globalement une forme complémentaire de l'espace interne délimité par lesdits éléments 14, 16 et 34 du moule 12 en position ouverte.

Les moyens 42 d'irradiation présentent par exemple une tête de forme permettant, en fonction du moule 12, de positionner lesdits moyens 42 d'irradiation à proximité notamment desdites surfaces 18, 36 de moulage.

En variante non représentée, les moyens 42 d'irradiation présentent une tête de forme globalement triangulaire pour un moule 12 de type portefeuille, de dimensions déterminées notamment en fonction de l'angle entre les éléments 14, 16 de moulage en position ouverte du moule 12, lesdites surfaces 18 de moulage à décontaminer n'étant pas coplanaires.

Dans le mode de réalisation, les lampes 44 sont fixes par rapport au premier support 50, en variante les lampes 44 sont montées mobiles pour permettre de régler leur position en fonction du moule 12 à décontaminer.

De préférence, les moyens 42 d'irradiation comportent un capot 84 destiné à protéger les lampes 44, ledit capot 84 s'étendant horizontalement au dessus des lampes 44 supérieures.

Avantageusement, les moyens 42 d'irradiation délimitent entre eux un logement destiné à être traversé verticalement par la tige 38 d'étirage du dispositif 10 de moulage, ledit logement permettant le passage de ladite tige 38 d'étirage.

De préférence, le capot 84 comporte une fente 86 longitudinale qui est située verticalement à l'aplomb du logement, soit de l'espace compris entre les lampes 44.

Avantageusement, le dispositif 40 de décontamination est apte à décontaminer également avec lesdits moyens 42 d'irradiation au moins une partie de la tige 38 d'étirage, en particulier l'extrémité libre de la tige 38 d'étirage destinée à entrer ultérieurement en contact avec le fond de la préforme au cours de la fabrication de récipients.

Avantageusement, des moyens réflecteurs sont associés auxdits moyens 42 d'irradiation pour focaliser le rayonnement ultraviolet émis en direction notamment desdites surfaces 18, 36 de moulage ou encore de la face 31 d'appui.

Les moyens réflecteurs sont par exemple constitués par un revêtement disposé en surface sur la face horizontale inférieure du capot 84 ou encore sur la partie du premier support 50 qui s'étend longitudinalement entre les lampes 44 formant les moyens 42 d'irradiation.

En variante non représentée, le dispositif 40 de décontamination comporte des moyens de nettoyage commandés qui sont aptes à être mis en oeuvre sélectivement pour nettoyer lesdits éléments 14, 16, 34 de moule, en particulier lesdites surfaces 18, 36 de moulage.

Avantageusement, les moyens de nettoyage consistent au moins à appliquer un agent nettoyant sur lesdits éléments 14, 16, 34 de moule et tout particulièrement sur au moins lesdites surfaces 18, 36 de moulage du moule 12 pour obtenir un moule propre, et cela préalablement à la décontamination par irradiation effectuée par les moyens 42 d'irradiation à rayonnement ultraviolet.

La désinfection est par exemple obtenue par application d'un agent nettoyant, présentant des propriétés germicides, sur l'ensemble des faces 30 interne desdits éléments 14, 16 de moule comportant les surfaces 18 de moulage ainsi que sur la surface 36 de moulage de l'élément 34 de fond de moule.

Avantageusement, ledit agent est appliqué directement sur au moins les surfaces 18, 36 de moulage par exemple par pulvérisation ou par l'intermédiaire de la brosse préalablement imbibée d'agent nettoyant.

De préférence, cette désinfection par voie chimique est mise en oeuvre en combinaison avec une action mécanique de brossage.

De préférence, les moyens de nettoyage comportent au moins une brosse qui est commandée en déplacement par des moyens d'entraînement associés entre une position de repos et une position de travail.

En position de travail, la brosse est agencée entre lesdits éléments 14, 16 et 34 du moule 12 et est par exemple entraînée en rotation afin de nettoyer lesdits éléments 14, 16 et 34 et tout particulièrement les surfaces 18, 36 de moulage avec lesquelles la brosse est en contact.

Avantageusement, l'agent nettoyant est à l'état liquide.

En variante, l'agent nettoyant est à l'état gazeux.

De préférence, l'agent nettoyant liquide est à base d'alcool ce qui présente l'avantage d'être sans risque (attaques chimiques, ...) pour les matériaux utilisés pour la réalisation du moule 12, voir plus généralement du dispositif 10 de moulage et d'une machine 102 de moulage, et qui permet d'obtenir un séchage rapide des surfaces traitées, sans traces résiduelles.

Avantageusement, le dispositif 40 de décontamination comporte donc des moyens 42 d'irradiation par rayonnement ultraviolet et des moyens de nettoyage.

Avantageusement, le dispositif 40 de décontamination est apte à décontaminer successivement les moules 12 des dispositifs 10 de moulage dont est munie une machine 102 de moulage du type de celle représentée à la figure 1 et décrite précédemment.

De préférence, le dispositif 40 de décontamination demeure à l'intérieur d'une enceinte 110 de protection que comporte ladite machine 102 et le dispositif 40 de décontamination est avantageusement implanté dans la zone Z matérialisée par des hachures sur la figure 1.

En mode de fonctionnement, dit de production, de la machine 102 et comme illustré sur la figure 1, les moules 12 occupent dans ladite zone Z la position fermée.

Pour permettre au dispositif 40 de décontamination selon le mode de réalisation représenté aux figures 3 et 4 d'intervenir sur le moule 12 d'un des dispositifs 10 de moulage dans la zone Z de la machine 102, l'ouverture du dispositif 10 de moulage doit préalablement être réalisée, puis le moule 12 occupant la position ouverte, les moyens 42 d'irradiation sont alors susceptibles d'être introduits entre les éléments 14, 16, 34 du moule 12 pour effectuer un cycle de décontamination.

Avantageusement, les opérations de déverrouillage et d'ouverture du dispositif 10 de moulage sont obtenues automatiquement et sélectivement avec un module de commande, lequel module est de préférence réalisé selon les enseignements du document EP-2.292.406 précité.

Selon ce document, le module de commande comporte un premier dispositif d'actionnement du dispositif de verrouillage (verrou 26) d'un dispositif 10 de moulage (ou "unité" selon la terminologie de ce document) et un deuxième dispositif d'actionnement pour commander l'ouverture et la fermeture du dispositif 10 de moulage.

En variante, l'ouverture automatique des moules 12 est obtenue automatiquement avec un module de commande réalisé conformément aux enseignements du document FR-2.954.207 auquel on se reportera pour une description détaillée de sa structure comme de son fonctionnement.

Grâce à un tel module de commande, le moule 12 d'un dispositif 10 de moulage peut être sélectivement ouvert dans la zone Z d'implantation du dispositif 40 de décontamination sans qu'un opérateur n'ait à y pénétrer.

On décrira maintenant un exemple de mise en oeuvre du dispositif 40 de décontamination selon le mode de réalisation décrit en référence aux figures 3 et 4, associé à une machine 102 de moulage implantée dans une installation 100 selon la figure 1.

L'invention propose encore un procédé de décontamination au moyen d'un dispositif 40 de décontamination comportant au moins des moyens 42 d'irradiation à rayonnement ultraviolet consistant à décontaminer au moins un moule 12 d'axe principal O qui est porté par un dispositif 10 de moulage équipant une machine 102 de moulage d'une installation 100 de fabrication de récipients à partir de préforme en matière thermoplastique et qui est monté mobile entre une position ouverte et une position fermée du moule.

Le procédé de décontamination comporte au moins :
- une étape (b0) de positionnement consistant à commander des moyens 46 d'entraînement associés aux moyens 42 d'irradiation à rayonnement ultraviolet pour introduire lesdits moyens 42 d'irradiation entre les éléments 14, 16, 34 du moule 12 occupant la position ouverte du moule ;
- une étape (b1) de décontamination consistant à irradier au moins lesdites surfaces 18, 36 de moulage desdits éléments 14, 16, 34 du moule 12 avec les moyens 42 d'irradiation à rayonnement ultraviolet.

De préférence, les moyens 42 d'irradiation à rayonnement ultraviolet sont introduits à l'intérieur du moule 12 suivant la direction longitudinale correspondant à l'axe X du trièdre (X, Y, Z), orthogonalement à l'axe principal 0 du moule 12.

L'étape (b0) de positionnement des moyens 42 d'irradiation du dispositif 40 de décontamination est précédée d'une étape consistant à commander la rotation du carrousel 106 de la machine 102 pour amener le dispositif 10 de moulage dans une position de référence déterminée par rapport au dispositif 40 de décontamination.

Grâce aux dispositifs d'actionnement d'un module de commande selon le document EP-2.292.406, le dispositif 10 de moulage est déverrouillé puis ouvert dans ladite zone Z d'implantation du dispositif 40 de décontamination de sorte que, dans ladite position de référence déterminée le moule 12 occupe sa position ouverte.

Avantageusement, aucune intervention d'un opérateur n'est requise pour réaliser le positionnement, le déverrouillage et l'ouverture du dispositif 10 de moulage, ces opérations étant réalisées automatiquement avec un tel module de commande, on réduit alors les risques de contamination, de l'environnement de fabrication en général et du moule 12 en particulier, qui sont liés toute présence humaine dans l'enceinte.

Selon l'étape (b0), les moyens 42 d'irradiation sont déplacés de la position de repos à la position de travail en pilotant les moyens 46 d'entraînement formés par les actionneurs 60, 66 et 70.

Le cycle de décontamination d'un moule 12 correspond par exemple aux opérations et/ou actions réalisées entre l'instant où les moyens 42 d'irradiation quittent la position de repos et celui où avantageusement ils reviennent dans cette position de repos.

En variante non représentée, les moyens 46 d'entraînement du dispositif 40 de décontamination sont simplifiés par rapport à l'exemple de réalisation des figures 3 et 4, la position intermédiaire pouvant notamment être supprimée.

Dans une telle variante, les moyens 46 d'entraînement comportent alors au moins un actionneur 66 pour déplacer longitudinalement lesdits moyens 42 d'irradiation selon l'axe X, respectivement entre une position de repos et une position de travail.

La position de repos correspondant à une position dans laquelle les moyens 42 d'irradiation sont en retrait par rapport au dispositif 10 de moulage comportant le moule 12 et la position de travail correspondant à une position dans laquelle les moyens 42 d'irradiation sont introduits à l'intérieur du moule 12 en position ouverte pour en décontaminer au moins lesdites surfaces 18, 36 de moulage.

Tel que décrit précédemment, les moyens 42 d'irradiation peuvent être fixes selon la direction verticale correspondant à l'axe Z du trièdre (X, Y, Z) ou comporter un actionneur pour les déplacer verticalement, notamment pour réaliser un balayage des surfaces 18, 36.

Par comparaison entre une telle variante et le mode de réalisation décrit précédemment en référence aux figures, la mobilité des moyens 42 d'irradiation selon la direction transversale correspondant à l'axe Y du trièdre (X, Y, Z) permet avantageusement au dispositif 40 de décontamination, en combinaison avec les moyens de positionnement, de se positionner automatiquement par rapport au moule 12 du dispositif 10 de moulage.

Avantageusement, le dispositif 10 de moulage est amené en position par rapport au dispositif 40 de décontamination du moule 12 sans toutefois nécessiter pour autant d'arrêter le carrousel 106 afin que le positionnement du dispositif 10 de moulage relativement au dispositif 40 soit très précis.

De préférence, le procédé de décontamination comporte une étape (a) de nettoyage du moule 12 qui est mise en oeuvre préalablement à l'étape (b1) de décontamination de manière que la décontamination par irradiation soit réalisée sur un moule propre.

Avantageusement, le procédé de décontamination du moule 12 comporte successivement au moins:
- une étape (a) de nettoyage consistant au moins à appliquer un agent nettoyant sur au moins lesdites surfaces de moulage du moule pour obtenir un moule propre ; et
- une étape (b1) de décontamination consistant à irradier au moins lesdites surfaces de moulage dudit moule propre au moyen des moyens d'irradiation à rayonnement ultraviolet.

L'étape (b1) de décontamination est en particulier précédée d'une d'étape (a) de nettoyage après qu'un changement des moules 12 ait été réalisé ou encore avant que ne débute à nouveau la fabrication de récipients après un arrêt de la machine 102 de moulage.

Lorsque des opérations de changement des moules d'une machine 102 sont effectuées, lesdites opérations sont alors avantageusement réalisées automatiquement conformément aux enseignements du document FR-2.972.385.

En variante, les opérations de changement des moules d'une machine 102 sont réalisées manuellement par au moins un opérateur.

De préférence, l'étape (a) de nettoyage consiste à mouiller au moins lesdites surfaces de moulage au moyen dudit agent nettoyant à l'état liquide et à frotter au moins lesdites surfaces de moulage pour obtenir un moule propre.

Avantageusement, l'étape (a) de nettoyage s'applique à l'ensemble de la surface desdits éléments 14, 16, 34 de moule, notamment la face 30 interne comme la face 31 d'appui.

Avantageusement, l'étape (a) de nettoyage est réalisée automatiquement par des moyens de nettoyage du dispositif 40 de décontamination du type de ceux décrits précédemment.

En variante, l'étape de nettoyage du moule 12 est effectuée manuellement par un opérateur

Toutefois, si l'étape (a) de nettoyage automatique ou manuelle est dans ce cas réalisée sur un moule 12 qui est monté sur un dispositif 10 de moulage, une telle étape (a) pourrait également en variante être mise en oeuvre sur un moule 12 démonté, en particulier avant le montage d'un tel moule 12 sur un dispositif 10 de moulage lors d'opérations de changement de moule de la machine 102.

Avantageusement, l'étape (a) de nettoyage mise en oeuvre sur un moule 12 avant son montage est ainsi réalisée en temps masqué par rapport aux autres opérations.

On comprendra que l'automatisation de l'ensemble des opérations précitées, avec ou sans changement de moule, en supprimant toute intervention d'au moins un opérateur permet de supprimer les risques de contamination liés à une présence humaine dans l'enceinte de la machine 102 de moulage d'une installation 100.

L'étape de nettoyage du moule 12, monté ou non sur un dispositif 10 de moulage, peut néanmoins être effectuée manuellement par un opérateur car l'étape (b1) de décontamination par irradiation permet d'éliminer ensuite les agents pathogènes qui seraient notamment présents sur les surfaces 18, 36 de moulage, y compris ceux résultant d'une contamination du moule 12 suite à l'intervention d'un opérateur.

L'étape (b0) de positionnement des moyens 42 d'irradiation consiste à commander les moyens 46 d'entraînement associés aux moyens 42 d'irradiation pour les déplacer de la position de repos représentée à la figure 5 à la position de travail représentée à la figure 6.

Avantageusement, la position de repos correspond à une position dans laquelle les moyens 42 d'irradiation du dispositif 40 de décontamination sont escamotés de manière à laisser le libre l'accès au moule 12 pour effectuer par exemple un changement du moule 12 ou encore l'étape (a) de nettoyage.

De plus, l'escamotage des moyens 42 d'irradiation en position de repos permet également d'éviter toute interférence avec les dispositifs 10 de moulage de la machine 102 lorsque la machine 102 est en mode de fonctionnement, dit de production.

Avantageusement, le dispositif 40 de décontamination comporte une unité de contrôle (non représentée) apte à commander les différents actionneurs 60, 66 et 70 et à les piloter grâce aux informations fournies notamment par les moyens de positionnement 78 et 80.

Pour déplacer les moyens 42 d'irradiation qui, en position de repos, sont protégés par le capot 76, l'unité commande tout d'abord le premier actionneur 60 pour provoquer la descente verticale selon l'axe Z du premier support 50 comportant les lampes 44 et cela jusqu'à ce que la tige 82 des deuxièmes moyens 80 de positionnement entre en contact avec la butée 88 portée par la pièce 75 sur laquelle est fixée le capot 76.

L'unité commande alors le troisième actionneur 70 pour déplacer transversalement selon l'axe Y les moyens 42 d'irradiation, les premiers moyens 78 de positionnement interviennent alors pour positionner les moyens 42 d'irradiation par rapport à l'axe O principal du moule 12 en position ouverte.

De préférence, le troisième actionneur étant un actionneur électrique, la course de déplacement transversal comporte une première course rapide en direction du moule 12 puis une course lente d'approche.

De préférence, l'unité commande le premier actionneur 60 pour déplacer les moyens 42 d'irradiation verticalement selon l'axe Z lors du déplacement transversal ou après celui-ci une fois lesdits moyens 42 d'irradiation centrés sur l'axe O du moule 12.

Grâce à un tel déplacement vertical, par exemple à une hauteur initiale correspondant à celle du capot 76, on prévient tout risque d'interférence entre lesdits moyens 42 d'irradiation et l'élément 34 de fond de moule.

En effet, l'unité commande ensuite le deuxième actionneur 66 pour introduire longitudinalement selon l'axe X lesdits moyens 42 d'irradiation dans le moule 12 ouvert, entre lesdits éléments 14, 16, 34 de moulage de manière à agencer lesdits moyens 42 d'irradiation à proximité au moins desdites surfaces 18, 36 de moulage.

Les moyens 42 d'irradiation occupent alors leur position de travail, toutefois ladite position de travail n'est avantageusement pas une position unique et fixe et cela tout particulièrement lorsque le moule 12 comporte un élément 34 de fond de moule.

De préférence, l'unité commande alors le premier actionneur 60 pour provoquer la descente des moyens 42 d'irradiation jusqu'à ce que la tige 82 des deuxièmes moyens 80 de positionnement détecte l'élément 34 de fond de moule.

La course de descente est alors arrêtée, les moyens 42 d'irradiation occupe la position initiale de travail dans laquelle les lampes 44 inférieures notamment sont agencées à proximité de la surface 36 de moulage de l'élément 34 de fond de moule.

L'unité commande alors le premier actionneur 60 pour que les moyens 42 d'irradiation effectue un déplacement vertical depuis cette position initiale de travail jusqu'à une position finale de travail, déplacement au cours duquel les lampes 44 vont irradier d'un rayonnement ultraviolet l'ensemble des faces 30 internes de chaque élément 14, 16 de moule et tout particulièrement les surfaces 18 de moulage et ce faisant décontaminer le moule 12.

Avantageusement, lorsque le dispositif 10 de moulage comporte une tige 38 d'étirage, ladite tige 38 est abaissée préalablement à l'introduction des moyens 42 d'irradiation à l'intérieur du moule 12 en position ouverte.

La tige 38 d'étirage est reçue dans le logement prévu à cet effet, entre les lampes 44, grâce à quoi au moins une partie de ladite tige 38 est également décontaminée.

En variante, la tige 38 d'étirage est abaissée vers le bas après la mise en place des moyens 42 d'irradiation en position de travail.

Pour déplacer les moyens 42 d'irradiation vers la position de repos, l'unité commande les actionneurs 60, 66 et 70 en sens inverse jusqu'à ramener lesdits moyens 42 dans le logement délimité par le capot 76 achevant alors au moins un cycle de décontamination.

Le procédé de décontamination détermine un cycle de décontamination d'un moule 12 qui est mis en oeuvre pour traiter successivement au moins une partie des moules 12 d'une machine 102 de moulage lorsque ladite machine 102 n'est pas en mode de fonctionnement, dit de production.

Avantageusement, le dispositif 40 de décontamination associé à la machine réalise successivement la décontamination de tout ou au moins une partie des différents moules 12 de la machine 102 de moulage de l'installation 100 suivant un cycle de décontamination tel que celui décrit précédemment.

De préférence, le retour dans ladite position de repos est effectué systématiquement après la décontamination de chaque moule 12 ou lorsque l'ensemble des moules 12 à décontaminer l'ont été.

En variante, les moyens 42 d'irradiation sont déplacés après la décontamination d'un moule 12, seulement de la position de travail à une position intermédiaire pour réduire le temps de cycle, en particulier en l'absence de risque d'inférences des moyens 42 d'irradiation du dispositif 40 de décontamination avec un dispositif 10 de moulage de la machine 102.

Le carrousel 106 de la machine 102 est entraîné en rotation pour amener le dispositif 10 de moulage suivant dans une position de référence déterminée par rapport au dispositif 40 de décontamination de manière à en décontaminer le moule 12.

## Revendications

1. Installation (100) pour la fabrication de récipients à partir de préformes en matière thermoplastique, ladite installation (100) comportant au moins :
- une machine (102) de moulage qui comporte au moins un dispositif (10) de moulage comportant un moule (12) ayant un axe principal (O), ledit moule (12) comportant au moins deux éléments (14, 16, 34) de moule pourvus chacun d'une surface (18, 36) de moulage et qui sont montés mobiles entre une position ouverte et une position fermée du moule, et
- un dispositif (40) de décontamination d'au moins un moule (12) porté par un dispositif (10) de moulage de la machine (102), ledit dispositif (40) de décontamination associé à la machine (102) comportant au moins des moyens (42) d'irradiation à rayonnement ultraviolet,
**caractérisée en ce que** ledit dispositif (40) de décontamination comporte des moyens (46) d'entraînement desdits moyens (42) d'irradiation à rayonnement ultraviolet pour introduire lesdits moyens (42) d'irradiation entre lesdits au moins deux éléments (14, 16, 34) de moule constitutifs du moule (12) du dispositif (10) de moulage,
lesdits moyens (46) d'entraînement étant commandés pour déplacer lesdits moyens (42) d'irradiation par rapport à la machine (102) entre au moins :
- une position de repos dans laquelle les moyens (42) d'irradiation sont escamotés pour éviter toute interférence entre lesdits moyens (42) d'irradiation du dispositif (40) de décontamination et le moule (12) du dispositif (10) de moulage de la machine (102), et au moins
- une position de travail dans laquelle les moyens (42) d'irradiation sont agencés à l'intérieur du moule (12) occupant sa position ouverte, entre lesdits éléments (14, 16, 34) du moule (12) pour décontaminer par irradiation au moins lesdites surfaces (18, 36) de moulage.

2. Installation selon la revendication 1, **caractérisée en ce que** lesdits moyens (46) d'entraînement déplacent dans l'espace selon un trièdre (X, Y, Z) lesdits moyens (42) d'irradiation pour les introduire, orthogonalement par rapport à l'axe (O) principal du moule (12), à l'intérieur du moule (12) en position ouverte.

3. Installation selon la revendication 1, **caractérisée en ce que** le dispositif (10) de moulage de la machine (102) comportant ledit moule (12) en position ouverte occupe une position fixe par rapport au dispositif (40) de décontamination au cours d'un cycle de décontamination par lesdits moyens (42) d'irradiation.

4. Installation selon l'une des revendications 1 à 3, **caractérisée en ce que** lesdits moyens (46) d'entraînement comportent au moins un actionneur (70) pour déplacer lesdits moyens (42) d'irradiation transversalement selon l'axe Y du trièdre (X, Y, Z) entre la position de repos et une position intermédiaire qui est déterminée par rapport à un axe (O) principal du moule.

5. Installation selon l'une quelconque des revendications 1 à 4, **caractérisée en ce que** lesdits moyens (46) d'entraînement comportent au moins un actionneur (66) pour déplacer lesdits moyens (42) d'irradiation longitudinalement selon l'axe X du trièdre (X, Y, Z) entre la position de repos ou ladite position intermédiaire et ladite position de travail.

6. Installation selon l'une quelconque des revendications 1 à 5, **caractérisée en ce que** lesdits moyens (46) d'entraînement comportent au moins un actionneur (60) pour déplacer lesdits moyens (42) d'irradiation verticalement selon l'axe Z du trièdre (X, Y, Z), notamment pour réaliser un balayage vertical des surfaces (18, 36) de moulage du moule.

7. Installation selon l'une quelconque des revendications 1 à 6, **caractérisée en ce que** des moyens (78, 80) de positionnement sont respectivement disposés sur le moule (12) et/ou sur le dispositif (40) de décontamination pour déterminer la position relative des moyens (42) d'irradiation par rapport au moule (12) et piloter lesdits moyens (46) d'entraînement associés aux moyens (42) d'irradiation à rayonnement ultraviolet.

8. Installation selon l'une quelconque des revendications précédentes, **caractérisée en ce que** lesdits moyens (42) d'irradiation sont activés au moins en position de travail.

9. Installation selon l'une quelconque des revendications précédentes, **caractérisée en ce que** les moyens (42) d'irradiation comportent entre eux un logement destiné à être traversé verticalement par une tige (38) d'étirage du dispositif (10) de moulage qui est associée audit moule (12) de manière à décontaminer au moins une partie de ladite tige (38) d'étirage avec lesdits moyens (42) d'irradiation.

10. Installation selon l'une quelconque des revendications précédentes, **caractérisée en ce que** des moyens réflecteurs sont associés auxdits moyens (42) d'irradiation pour focaliser le rayonnement ultraviolet émis en direction au moins desdites surfaces (18, 36) de moulage.

11. Installation selon l'une quelconque des revendications précédentes, **caractérisée en ce que** le dispositif (40) de décontamination comporte des moyens de nettoyage qui sont commandés sélectivement pour nettoyer au moins lesdites surfaces (18, 36) de moulage.

12. Procédé de décontamination au moyen d'un dispositif (40) de décontamination comportant au moins des moyens (42) d'irradiation à rayonnement ultraviolet consistant à décontaminer au moins un moule (12) d'axe principal (O) qui est porté par un dispositif (10) de moulage équipant une machine (102) de moulage d'une installation (100) de fabrication de récipients à partir de préforme en matière thermoplastique et qui est monté mobile entre une position ouverte et une position fermée du moule, **caractérisé en ce que** ledit procédé de décontamination comporte au moins successivement :
- une étape (b0) de positionnement consistant à commander des moyens (46) d'entraînement associés aux moyens (42) d'irradiation à rayonnement ultraviolet pour introduire, orthogonalement à l'axe (O) principal du moule (12), lesdits moyens (42) d'irradiation entre lesdits au moins deux éléments (14, 16, 34) du moule (12) occupant ladite position ouverte du moule ;
- une étape (b1) de décontamination consistant à irradier au moins lesdites surfaces (18, 36) de moulage desdits éléments (14, 16, 34) du moule (12) avec lesdits moyens (42) d'irradiation à rayonnement ultraviolet.

13. Procédé selon la revendication 12, **caractérisé en ce que** ledit procédé de décontamination comporte avant lesdites étapes (b0) et (b1):
- une étape (a) de nettoyage consistant au moins à appliquer un agent nettoyant sur au moins lesdites surfaces (18, 36) de moulage du moule (12) pour obtenir un moule propre.

14. Procédé selon l'une des revendications 12 ou 13, **caractérisé en ce que** ledit procédé de décontamination détermine un cycle de décontamination d'un moule (12) qui est mis en oeuvre pour traiter successivement au moins une partie des moules (12) d'une machine (102) de moulage lorsque ladite machine (102) n'est pas en mode de fonctionnement, dit de production.

## Patentansprüche

1. Anlage (100) zur Herstellung von Behältern ausgehend von Vorformlingen aus thermoplastischem Material, wobei die Anlage (100) mindestens aufweist:
- eine Formgebungsmaschine (102), die mindestens eine Formgebungsvorrichtung (10) aufweist, welche eine Form (12) mit einer Hauptachse (0) aufweist, wobei die Form (12) mindestens zwei Formelemente (14, 16, 34) aufweist, die je mit einer Formgebungsfläche (18, 36) versehen und zwischen einer offenen und einer geschlossenen Stellung der Form beweglich sind, und
- eine Vorrichtung (40) zur Dekontaminierung mindestens einer von einer Formgebungsvorrichtung (10) der Maschine (102) getragenen Form (12), wobei die der Maschine (102) zugeordnete Dekontaminierungsvorrichtung (40) mindestens UV-Bestrahlungseinrichtungen (42) aufweist,
**dadurch gekennzeichnet, dass** die Dekontaminierungsvorrichtung (40) Antriebseinrichtungen (46) der UV-Bestrahlungseinrichtungen (42) aufweist, um die Bestrahlungseinrichtungen (42) zwischen die mindestens zwei Formelemente (14, 16, 34) einzuführen, die die Form (12) der Formgebungsvorrichtung (10) bilden,
wobei die Antriebseinrichtungen (46) gesteuert werden, um die Bestrahlungseinrichtungen (42) bezüglich der Maschine (102) zu verschieben zwischen mindestens:
- einer Ruhestellung, in der die Bestrahlungseinrichtungen (42) eingezogen sind, um jede Wechselwirkung zwischen den Bestrahlungseinrichtungen (42) der Dekontaminierungsvorrichtung (40) und der Form (12) der Formgebungsvorrichtung (10) der Maschine (102) zu verhindern, und mindestens
- einer Arbeitsstellung, in der die Bestrahlungseinrichtungen (42) im Inneren der Form (12), die ihre offene Stellung einnimmt, zwischen den Elementen (14, 16, 34) der Form (12) angeordnet sind, um mindestens die Formgebungsflächen (18, 36) durch Bestrahlung zu dekontaminieren.

2. Anlage nach Anspruch 1, **dadurch gekennzeichnet, dass** die Antriebseinrichtungen (46) die Bestrahlungseinrichtungen (42) im Raum gemäß einem Koordinatensystem (X, Y, Z) verschieben, um sie orthogonal bezüglich der Hauptachse (0) der Form (12) ins Innere der Form (12) in offener Stellung einzuführen.

3. Anlage nach Anspruch 1, **dadurch gekennzeichnet, dass** die Formgebungsvorrichtung (10) der Maschine (102), die die Form (12) in offener Stellung aufweist, während eines Dekontaminierungszyklus durch die Bestrahlungseinrichtungen (42) eine bezüglich der Dekontaminierungsvorrichtung (40) ortsfeste Stellung einnimmt.

4. Anlage nach einem der Ansprüche 1 bis 3, **dadurch gekennzeichnet, dass** die Antriebseinrichtungen (46) mindestens einen Stellantrieb (70) aufweisen, um die Bestrahlungseinrichtungen (42) gemäß der Y-Achse des Koordinatensystems (X, Y, Z) zwischen der Ruhestellung und einer Zwischenstellung quer zu verschieben, die bezüglich einer Hauptachse (0) der Form bestimmt wird.

5. Anlage nach einem der Ansprüche 1 bis 4, **dadurch gekennzeichnet, dass** die Antriebseinrichtungen (46) mindestens einen Stellantrieb (66) aufweisen, um die Bestrahlungseinrichtungen (42) gemäß der X-Achse des Koordinatensystems (X, Y, Z) wischen der Ruhestellung oder der Zwischenstellung und der Arbeitsstellung in Längsrichtung zu verschieben.

6. Anlage nach einem der Ansprüche 1 bis 5, **dadurch gekennzeichnet, dass** die Antriebseinrichtungen (46) mindestens einen Stellantrieb (60) aufweisen, um die Bestrahlungseinrichtungen (42) gemäß der Z-Achse des Koordinatensystems (X, Y, Z) senkrecht zu verschieben, insbesondere, um eine senkrechte Abtastung der Formgebungsflächen (18, 36) der Form durchzuführen.

7. Anlage nach einem der Ansprüche 1 bis 6, **dadurch gekennzeichnet, dass** Positioniereinrichtungen (78, 80) auf der Form (12) und/oder auf der Dekontaminierungsvorrichtung (40) angeordnet sind, um die relative Stellung der Bestrahlungseinrichtungen (42) bezüglich der Form (12) zu bestimmen und die den UV-Bestrahlungseinrichtungen (42) zugeordneten Antriebseinrichtungen (46) zu steuern.

8. Anlage nach einem der vorhergehenden Ansprüche, **dadurch gekennzeichnet, dass** die Bestrahlungseinrichtungen (42) zumindest in der Arbeitsstellung aktiviert werden.

9. Anlage nach einem der vorhergehenden Ansprüche, **dadurch gekennzeichnet, dass** die Bestrahlungseinrichtungen (42) zwischen sich eine Aufnahme aufweisen, die dazu bestimmt ist, senkrecht von einer Reckstange (38) der Formgebungsvorrichtung (10) durchquert zu werden, die der Form (12) zugeordnet ist, um mindestens einen Teil der Reckstange (38) mit den Bestrahlungseinrichtungen (42) zu dekontaminieren.

10. Anlage nach einem der vorhergehenden Ansprüche, **dadurch gekennzeichnet, dass** Reflektoreinrichtungen den Bestrahlungseinrichtungen (42) zugeordnet sind, um die emittierte UV-Strahlung mindestens in Richtung der Formgebungsflächen (18, 36) zu fokussieren.

11. Anlage nach einem der vorhergehenden Ansprüche, **dadurch gekennzeichnet, dass** die Dekontaminierungsvorrichtung (40) Reinigungseinrichtungen aufweist, die selektiv gesteuert werden, um mindestens die Formgebungsflächen (18, 36) zu reinigen.

12. Verfahren zur Dekontaminierung mittels einer Dekontaminierungsvorrichtung (40), die mindestens UV-Bestrahlungseinrichtungen (42) aufweist, das darin besteht, mindestens eine Form (12) mit einer Hauptachse (0) zu dekontaminieren, die von einer Formgebungsvorrichtung (10) getragen wird, welche zu einer Formgebungsmaschine (102) einer Anlage (100) zur Herstellung von Behältern ausgehend von Vorformlingen aus thermoplastischem Material gehört, und die zwischen einer offenen Stellung und einer geschlossenen Stellung der Form beweglich montiert ist, **dadurch gekennzeichnet, dass** das Dekontaminierungsverfahren mindestens nacheinander aufweist:
- einen Positionierungsschritt (b0), der darin besteht, den UV-Bestrahlungseinrichtungen (42) zugeordnete Antriebseinrichtungen (46) zu steuern, um orthogonal zur Hauptachse (0) der Form (12) die Bestrahlungseinrichtungen (42) zwischen die mindestens zwei Elemente (14, 16, 34) der Form (12) einzuführen, die die offene Stellung der Form einnimmt;
- einen Dekontaminierungsschritt (b1), der darin besteht, mindestens die Formgebungsflächen (18, 36) der Elemente (14, 16, 34) der Form (12) mit den UV-Bestrahlungseinrichtungen (42) zu bestrahlen.

13. Verfahren nach Anspruch 12, **dadurch gekennzeichnet, dass** das Dekontaminierungsverfahren vor den Schritten (b0) und (b1) aufweist:
- einen Reinigungsschritt (a), der mindestens darin besteht, ein Reinigungsmittel auf mindestens die Formgebungsflächen (18, 36) der Form (12) aufzubringen, um eine saubere Form zu erhalten.

14. Verfahren nach einem der Ansprüche 12 oder 13, **dadurch gekennzeichnet, dass** das Dekontaminierungsverfahren einen Dekontaminierungszyklus einer Form (12) bestimmt, der durchgeführt wird, um nacheinander mindestens einen Teil der Formen (12) einer Formgebungsmaschine (102) zu behandeln, wenn die Maschine (102) nicht im sogenannten Produktions-Betriebsmodus ist.

## Claims

1. Installation (100) for manufacturing containers from preforms made of thermoplastic material, said installation (100) comprising at least:
- one molding machine (102) which comprises at least one molding device (10) comprising a mold (12) having a main axis (0), said mold (12) comprising at least two mold elements (14, 16, 34) each provided with a molding surface (18, 36) and which are mounted to move between an open position and a closed position of the mold, and
- a device (40) for decontaminating at least one mold (12) borne by a molding device (10) of the machine (102), said decontamination device (40) associated with the machine (102) comprising at least ultraviolet radiation irradiation means (42),
**characterized in that** said decontamination device (40) comprises means (46) for driving said ultraviolet radiation irradiation means (42) to introduce said irradiation means (42) between said at least two constituent mold elements (14, 16, 34) of the mold (12) of the molding device (10),
said driving means (46) being controlled to displace said irradiation means (42) relative to the machine (102) between at least:
- a rest position in which the irradiation means (42) are retracted to avoid any interference between said irradiation means (42) of the decontamination device (40) and the mold (12) of the molding device (10) of the machine (102), and at least
- a working position in which the irradiation means (42) are arranged inside the mold (12) occupying its open position, between said elements (14, 16, 34) of the mold (12) to decontaminate by irradiation at least said molding surfaces (18, 36).

2. Installation according to Claim 1, **characterized in that** said driving means (46) displace, in the space according to a trihedron (X, Y, Z), said irradiation means (42) to introduce them, orthogonally relative to the main axis (0) of the mold (12), inside the mold (12) in the open position.

3. Installation according to Claim 1, **characterized in that** the molding device (10) of the machine (102) comprising said mold (12) in the open position occupies a fixed position relative to the decontamination device (40) during a cycle of decontamination by said irradiation means (42).

4. Installation according to one of Claims 1 to 3, **characterized in that** said driving means (46) comprise at least one actuator (70) for displacing said irradiation means (42) transversely along the Y axis of the trihedron (X, Y, Z) between the rest position and an intermediate position which is determined relative to a main axis (0) of the mold.

5. Installation according to any one of Claims 1 to 4, **characterized in that** said driving means (46) comprise at least one actuator (66) for displacing said irradiation means (42) longitudinally along the X axis of the trihedron (X, Y, Z) between the rest position or said intermediate position and said working position.

6. Installation according to any one of Claims 1 to 5, **characterized in that** said driving means (46) comprise at least one actuator (60) for displacing said irradiation means (42) vertically along the Z axis of the trihedron (X, Y, Z), notably to perform a vertical sweep of the molding surfaces (18, 36) of the mold.

7. Installation according to any one of Claims 1 to 6, **characterized in that** positioning means (78, 80) are respectively arranged on the mold (12) and/or on the decontamination device (40) to determine the relative position of the irradiation means (42) relative to the mold (12) and drive said driving means (46) associated with the ultraviolet radiation irradiation means (42).

8. Installation according to any one of the preceding claims, **characterized in that** said irradiation means (42) are activated at least in the working position.

9. Installation according to any one of the preceding claims, **characterized in that** the irradiation means (42) comprise, between them, a housing intended to be passed through vertically by a drawing rod (38) of the molding device (10) which is associated with said mold (12) so as to decontaminate at least a part of said drawing rod (38) with said irradiation means (42).

10. Installation according to any one of the preceding claims, **characterized in that** reflector means are associated with said irradiation means (42) to focus the ultraviolet radiation emitted toward at least said molding surfaces (18, 36).

11. Installation according to any one of the preceding claims, **characterized in that** the decontamination device (40) comprises cleaning means which are selectively controlled to clean at least said molding surfaces (18, 36).

12. Decontamination method by means of a decontamination device (40) comprising at least ultraviolet radiation irradiation means (42) consisting in decontaminating at least one mold (12) of main axis (0) which is borne by a molding device (10) with which a molding machine (102) of an installation (100) for manufacturing containers from preforms made of thermoplastic material is equipped, and which is mounted to move between an open position and a closed position of the mold, **characterized in that** said decontamination method comprises at least, in succession:
- a positioning step (b0) consisting in controlling driving means (46) associated with the ultraviolet radiation irradiation means (42) to introduce, orthogonally to the main axis (0) of the mold (12), said irradiation means (42) between said at least two elements (14, 16, 34) of the mold (12) occupying said open position of the mold;
- a decontamination step (b1) consisting in irradiating at least said molding surfaces (18, 36) of said elements (14, 16, 34) of the mold (12) with said ultraviolet radiation irradiation means (42).

13. Method according to Claim 12, **characterized in that** said decontamination method comprises, before said steps (b0) and (b1) :
- a cleaning step (a) consisting at least in applying a cleaning agent to at least said molding surfaces (18, 36) of the mold (12) to obtain a clean mold.

14. Method according to either of Claims 12 and 13, **characterized in that** said decontamination method determines a mold decontamination cycle (12) which is implemented to successively treat at least some of the molds (12) of a molding machine (102) when said machine (102) is not in so-called production operating mode.
